# EUROPEAN PATENT APPLICATION

(11) **EP 4 534 152 A1**
(43) Date of publication of application: **09.04.2025**
(21) Application number: 23815947.9
(22) Date of filing: 26.05.2023
(51) Int. Cl.: A61Q 19/00, A61Q 19/10, A61Q 5/00, A61Q 5/02, A61Q 5/12, A61K 8/02, A61K 8/72, A61K 8/73, A61K 8/81, A61K 8/86

(54) **AQUEOUS COMPOSITION FOR BODY**

(30) Priority: 30.05.2022 JP 2022087738; 30.05.2022 JP 2022087749; 24.02.2023 JP 2023027590
(71) Applicant: Kao Corporation, Tokyo 103-8210 (JP)
(72) Inventor: KAWAMOTO Koichi, Tokyo 131-8501 (JP); SARUKAWA Yuri, Tokyo 131-8501 (JP); MIYOSHI Eisuke, Tokyo 131-8501 (JP)
(74) Representative: Hoffmann Eitle
(86) International application number: PCT/JP2023/019639
(87) International publication number: WO 2023/234190

(57) **Abstract**

An aqueous composition for body, the aqueous composition containing (A) a cationic polymer having a viscosity when in a 1 mass% aqueous solution form at 30°C of 1000 mPa·s or more, (B) an anionic polymer, (C) one or more selected from the group consisting of (C1) a nonionic polymer and (C2) agar, and water.

## Description

### Technical Field

The present invention relates to an aqueous composition for body.

Aqueous compositions for treating keratin materials containing cationic polymers and anionic polymers are known. For example, JP 53-139734 A (PTL 1) describes that using a composition for treating a keratin material, which is characterized by containing at least one anionic polymer and at least one cationic polymer in a solvent medium, makes it possible to fix the anionic polymer to a keratin material that can particularly include hair, skin, or nails. It is assumed that a complex formed by an interaction between the anionic polymer and the cationic polymer is related to the reason for the above-described effect.

Regarding hair cosmetics containing a cationic polymer and an anionic polymer, JP H10-287535 A (PTL 2) describes that a semipermanent hair dye composition containing one or two or more polymer compounds selected from the group consisting of an aromatic alcohol, a cationic compound, and a water-soluble anionic polymer compound, a direct dye, and an acid and having a value of the pH and the viscosity equal to or larger than a predetermined value has a sufficient dyeing property and a high conditioning effect and has a stable viscosity at a high temperature or over a long period of time.

JP 2004-525101 A (PTL 3) discloses a composition containing, in a physiologically acceptable medium including a fatty phase: (i) a first polymer having a weight average molecular mass of less than 100000, including a) a polymer backbone having hydrocarbon-based repeat units including at least one heteroatom, and optionally b) a pendant and/or terminal fatty chain optionally functionalized, including from 6 to 120 carbon atoms, bonded to these hydrocarbon-based units; (ii) an anionic film-forming polymer; and (iii) a cationic film-forming polymer. It is described that this composition provides a quick makeup effect to the keratin substance, and is applied to a makeup method and care for the keratin substance, in particular, as mascara.

JP 2006-70030 A (PTL 4) describes that a hairstyling gel containing a combination of a first polymer composed of a hydrocolloid based on a nonionic or anionic polysaccharide, a second polymer composed of an acryl- or methacrylamidoalkylsulfonic acid or a salt thereof, and a third polymer that is an amphiphilic polymer exhibits a volume-imparting effect on wet hair and has an effect of structuring a hairstyle on dry hair.

JP 2015-166333 (PTL 5) describes that a hair cosmetic containing a cationic polymer having a charge density in a predetermined range and an anionic polymer containing 80 mass% or more and 100 mass% or less of a predetermined anionic constituent unit and having a weight average molecular weight of 4000 or more and 80000 or less can improve the texture and appearance of damaged hair, and these effects can be maintained even by repeated washing of hair.

### Summary of Invention

The present invention relates to the following.
[1] An aqueous composition for body, the aqueous composition containing (A) a cationic polymer having a viscosity when in a 1 mass% aqueous solution form at 30°C of 1000 mPa·s or more, (B) an anionic polymer, (C) one or more selected from the group consisting of (C1) a nonionic polymer and (C2) agar, and water.
[2] A cosmetic composition containing the aqueous composition for body described in [1].
[3] A hair treatment method including applying the cosmetic composition described in [2] to hair, and then heating the hair to a temperature of 80°C or more.

### Description of Embodiments

As described in PTL 1 or the like, it is known to use an aqueous composition containing a complex formed from a cationic polymer and an anionic polymer (hereinafter, also referred to as "polyion complex") for a composition for treating a keratin substance such as hair.

The polyion complex is an aggregate formed by ionic interaction between a cationic polymer and an anionic polymer, and when a treatment composition containing the polyion complex is applied to an object to be treated, a cuticle-like high elastic film can be formed. Thus, the texture of the object to be treated is improved. For example, when the composition is used as a hair cosmetic composition such as a hair conditioner, a hair care effect is exhibited, and an improvement in the effect of suppressing entanglement of hair is expected. However, PTLs 1 to 5 do not particularly refer to improvement in the effect of suppressing hair entanglement. In addition, the hair treatment effect may be lost by one time of hair washing, and the durability (washing resistance) of the treatment effect has been desired.

The present invention relates to an aqueous composition for body, which can improve a texture improving effect of an object to be treated and durability of the effect, and particularly can improve an effect of suppressing entanglement of hair and durability thereof when used for hair treatment.

The present inventors have found that the above problem can be solved by an aqueous composition containing a cationic polymer having a viscosity of equal to or larger than a predetermined value when in a 1 mass% aqueous solution form, an anionic polymer, one or more selected from the group consisting of a nonionic polymer and agar, and water.

According to the present invention, it is possible to provide an aqueous composition for body that can improve the effect of improving the texture of an object to be treated and the durability of the effect, and in particular, can improve the effect of suppressing entanglement of hair and the durability thereof when used for hair treatment.

The aqueous composition can be used as various cosmetic compositions such as a skin cosmetic composition and a hair cosmetic composition. The aqueous composition can also be used as a premix for various cosmetic compositions. For example, additional components may be added to the aqueous composition to prepare a skin cosmetic composition or a hair cosmetic composition.

### [Definitions]

**In** the present specification, "aqueous composition for body" means an aqueous composition used for treating a surface of the body, for example, a surface of a keratin substance such as skin, hair, eyelashes, eyebrows, or nails. The term "aqueous composition" means a composition containing water as a main component, and preferably means a composition having a water content of 50 mass% or more.

The aqueous composition for body of the present invention is preferably an aqueous composition for treating keratin materials, and more preferably an aqueous composition for treating skin or hair.

**In** the present specification, "containing a component X" also includes blending of a component X.

### [Aqueous Composition for Body]

The aqueous composition for body of the present invention (hereinafter also simply referred to as "aqueous composition" or "composition of the present invention") contains (A) a cationic polymer having a viscosity when in a 1 mass% aqueous solution form at 30°C of 1000 mPa·s or more, (B) an anionic polymer, (C) one or more selected from the group consisting of (C1) a nonionic polymer and (C2) agar.

The composition of the present invention, having the above configuration, can improve the effect of improving the texture of an object to be treated and the durability of the effect, and in particular, can improve the effect of suppressing entanglement of hair and the durability thereof when used for hair treatment.

The reason why the composition of the present invention exhibits the effects described above is not clear, but the reason is thought to be as follows.

The composition of the present invention contains a polyion complex formed by ionic interaction between the component (A) and the component (B). In the polyion complex, a crosslinked structure is formed by an ionic interaction between the component (A) and the component (B), and it is assumed that this structure causes the film obtained by using the composition of the present invention to exhibit a high elastic modulus.

The composition of the present invention further contains one or more selected from the group consisting of (C1) a nonionic polymer and (C2) agar as the component (C).

In the film obtained by using the composition containing (C1) a nonionic polymer as the component (C), it is considered that the rigid crosslinked structure of the polyion complex formed by the component (A) and the component (B) is reinforced by the complexation with the long-chain and flexible non-crosslinked structure of the component (C1). Since a hydrogen bond can be formed between the component (B) and the component (C1), it is considered that the component (C1) is held in the crosslinked structure by the component (B), and a network having higher strength is formed in the film. Thus, it is considered that when the composition of the present invention is used, an effect of improving the texture of an object to be treated or the like is obtained even by one treatment, and the treatment effect is not lost even when washing is performed thereafter, and the durability of the effect is obtained.

In the film obtained by using the composition containing (C2) agar as the component (C), it is considered that the crosslinked structure formed by the component (A) and the component (B) is reinforced by the component (C2) having a double helix structure. Since hydrogen bond can be formed between the component (B) and the component (C2), the component (C2) is retained in the crosslinked structure by the component (B). Further, since the component (C2) undergoes sol-gel transition through heating and becomes a hard gel having a three-dimensional crosslinked structure when cooled, it is considered that a network having higher strength composed of the component (A), the component (B), and the component (C2) is formed in the film.

It is considered that when the viscosity of a 1 mass% aqueous solution of the component (A) used in the present invention is 1000 mPa·s or more at 30°C, the lubricity of the resulting film is improved. As a result, the texture of the object to be treated further improves, and it is considered that the effect of suppressing hair entanglement improves when the composition of the present invention is used for hair treatment.

### (Component (A): Cationic Polymer)

The component (A) is a cationic polymer having a viscosity when in a 1 mass% aqueous solution form at 30°C of 1000 mPa·s or more, and is a polymer capable of forming a polyion complex through interaction with the component (B).

The cationic polymer as the component (A) is preferably a polymer having a cationic group and being positively charged as a total charge. The component (A) may have an anionic group, a nonionic group, or an amphoteric group such as a betaine group in addition to the cationic group as long as the effects of the present invention are not impaired.

In the present specification, the cationic group is a cationic group or a group that can be ionized to become a cationic group, and specific examples thereof include a primary amino group, a secondary amino group, a tertiary amino group, and a quaternary ammonium group.

The anionic group is an anionic group or a group that can be ionized to become an anionic group, and specific examples thereof include one or more selected from the group consisting of acidic groups such as a carboxy group, a sulfonate group, and a phosphate group, preferably one or more selected from a carboxy group and a sulfonate group, and more preferably a carboxy group. At least a part of the anionic groups may be neutralized to form a salt.

The viscosity of a 1 mass% aqueous solution of the component (A) at 30°C is 1000 mPa·s or more, preferably 1300 mPa·s or more, more preferably 1500 mPa·s or more, even more preferably 1700 mPa·s or more, and even more preferably 1800 mPa·s or more, from the viewpoint of the texture improving effect of the object to be treated and the improvement of the durability of the effect, in particular, from the viewpoint of the effect of suppressing entanglement of hair and improving the durability thereof when used for hair treatment. The upper limit of the viscosity is not particularly limited, but is preferably 100000 mPa·s or less, more preferably 80000 mPa·s or less, even more preferably 50000 mPa·s or less, even more preferably 30000 mPa·s or less, and even more preferably 25000 mPa·s or less, from the viewpoint of ease of forming a polyion complex and handleability. The viscosity of a 1 mass% aqueous solution of the component (A) at 30°C is 1000 mPa·s or more, preferably 1000 mPa·s or more and 100000 mPa·s or less, more preferably 1300 mPa·s or more and 80000 mPa·s or less, even more preferably 1500 mPa·s or more and 50000 mPa·s or less, even more preferably 1700 mPa·s or more and 30000 mPa·s or less, and even more preferably 1800 mPa·s or more and 25000 mPa·s or less.

The viscosity at 30°C of a 1 mass% aqueous solution of the component (A) means the viscosity at 30°C of an aqueous solution in which the amount of the active ingredient of the cationic polymer is 1 mass%, and specifically, the viscosity can be measured by the method described in Examples.

The cationic charge density of the component (A) is preferably 0.1 mmol/g or more, more preferably 0.2 mmol/g or more, even more preferably 0.3 mmol/g or more, and even more preferably 0.5 mmol/g or more, from the viewpoint of facilitating formation of the polyion complex through interaction with the component (B), improving the texture of the object to be treated and the durability of the effect, in particular, the effect of inhibiting entanglement of hair and improving the durability thereof when used for hair treatment. The cationic charge density is preferably 20 mmol/g or less, more preferably 15 mmol/g or less, even more preferably 12 mmol/g or less, even more preferably 10 mmol/g or less, even more preferably 7.0 mmol/g or less, and even more preferably 5.0 mmol/g or less from the viewpoints of improving the texture of the object to be treated and improving the durability of the effect, in particular, suppressing the entanglement of hair and improving the durability thereof when used for hair treatment. The cationic charge density of the component (A) is preferably 0.1 mmol/g or more and 20 mmol/g or less, more preferably 0.2 mmol/g or more and 15 mmol/g or less, 0.2 mmol/g or more and 12 mmol/g or less, even more preferably 0.3 mmol/g or more and 10 mmol/g or less, even more preferably 0.3 mmol/g or more and 7.0 mmol/g or less, and even more preferably 0.5 mmol/g or more and 5.0 mmol/g or less.

The cationic charge density of the component (A) is the number of moles of cationic groups contained per polymer 1 g. When at least a part of the cationic groups of the component (A) is in the form of a neutralized salt, the number of moles of the cationic groups includes the number of moles of the cationic groups in the form of a salt.

Two or more polymers may be used as the component (A), and in this case, the cationic charge density of the component (A) is determined by calculating a weighted average from the cationic charge density and the blending amount of each polymer.

As the component (A), any of a natural polymer such as a cationized polysaccharide or a cationized product thereof and a synthetic polymer can be used, but from the viewpoint of availability, a synthetic polymer is preferable.

Specific examples of the polymer used as the component (A) include cationized polyvinyl alcohols, polyethyleneimines, methacryloylethyltrimethylammonium salt polymers, quaternized dialkylaminoalkyl (meth)acrylic acid salt polymers, diallylquaternized ammonium salts, methacrylamidopropyltrimethylammonium salt polymers, vinylimidazoliumtrichloride-vinylpyrrolidone copolymers (polyquaternium-16), vinylpyrrolidone-alkylamino (meth)acrylate copolymers, vinylpyrrolidone-alkylamino (meth)acrylate-vinylcaprolactam copolymers, alkylacrylamide-(meth)acrylate-alkylaminoalkylacrylamide-polyethylene glycol (meth)acrylate copolymers, and adipic acid-dimethylaminohydroxypropylethylenetriamine copolymers having a viscosity when in a 1 mass% aqueous solution form at 30°C of 1000 mPa·s or more, and one or two or more of these can be used. In the present specification, "(meth)acrylic acid" means acrylic acid or methacrylic acid, and "(meth)acrylate" means acrylate or methacrylate.

Among these, examples of the methacryloylethyltrimethylammonium salt polymer include a methacryloylethyltrimethylammonium chloride polymer (polyquaternium-37), a methacryloylethyldimethylbetaine-methacryloylethyltrimethylammonium chloride-methoxypolyethylene glycol methacrylate copolymer (polyquaternium-49), and a methacryloylethyldimethylbetaine-methacryloylethyltrimethylammonium chloride-2-hydroxyethyl methacrylate copolymer (polyquaternium-48).

Examples of the quaternized dialkylaminoalkyl (meth)acrylic acid salt polymer include a vinylpyrrolidone-N,N-dimethylaminoethyl methacrylate diethyl sulfate copolymer (polyquaternium-11) and an N,N-dimethylaminoethyl methacrylate diethyl sulfate-N,N-dimethylacrylamide-polyethylene glycol dimethacrylate copolymer (polyquaternium-52).

Examples of the diallyl quaternized ammonium salt polymer include a dimethyldiallylammonium chloride polymer (polyquaternium-6), a dimethyldiallylammonium chloride-acrylic acid copolymer (polyquaternium-22), a dimethyldiallylammonium chloride-acrylamide copolymer (polyquaternium-7), and an acrylamide-acrylic acid-dimethyldiallylammonium chloride copolymer (polyquaternium-39).

Examples of the methacrylamidopropyltrimethylammonium salt polymer include a methacrylamidopropyltrimethylammonium chloride polymer, a vinylpyrrolidone-methacrylamidopropyltrimethylammonium chloride copolymer, an acrylic acid-methyl acrylate-methacrylamidopropyltrimethylammonium chloride copolymer (polyquaternium-47), and an acrylic acid-acrylamide-methacrylamidopropyltrimethylammonium chloride copolymer (polyquaternium-53).

The component (A) is preferably a polymer containing a constituent unit represented by the following General Formula (1) from the viewpoint of improving the film-forming ability, the texture of the object to be treated, the effect of improving the texture, and the durability of the effect, in particular, improving the effect of suppressing entanglement of hair and improving the durability thereof when used for hair treatment. 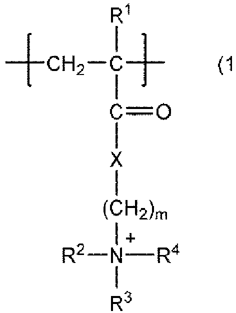 wherein R¹ is a hydrogen atom or a methyl group, and R² to R⁴ are each independently an alkyl group having 1 or more and 4 or less carbons, X is -O- or -NH-, and m is the number of 1 to 4.

In Formula (1), R¹ is preferably a methyl group, and R² to R⁴ are each independently preferably an alkyl group having 1 or more and 3 or less carbons, more preferably a methyl group or an ethyl group, and even more preferably a methyl group.

X in Formula (1) is preferably -O-, and m is preferably 1 or more and 3 or less, and more preferably 2 or more and 3 or less.

Examples of the constituent unit represented by Formula (1) include one or more selected from the group consisting of a constituent unit derived from a methacryloylethyltrimethylammonium salt, a constituent unit derived from N,N-dimethylaminoethylmethacrylic acid diethyl sulfate, and a constituent unit derived from a methacrylamidopropyltrimethylammonium salt, and preferably one or more selected from the group consisting of a constituent unit derived from a methacryloylethyltrimethylammonium salt and a constituent unit derived from N,N-dimethylaminoethylmethacrylic acid diethyl sulfate.

The component (A) may be a polymer containing an additional constituent unit other than the constituent unit represented by Formula (1). Examples of the additional constituent unit include constituent units derived from vinyl monomers such as vinylpyrrolidone, amphoteric monomers such as (meth)acryloylethyldimethylbetaine, hydroxyalkyl (meth)acrylates such as (meth)acrylic acid alkyl esters and 2-hydroxyethyl (meth)acrylate, and acrylamides such as N,N-dimethylacrylamide. The component (A) may also be a crosspolymer crosslinked with di(meth)acrylic acid polyethylene glycol or the like.

In the component (A), the content of the constituent unit represented by General Formula (1) is preferably 8.0 mol% or more and 100 mol% or less, based on all constituent units constituting the component (A), from the viewpoint of improving the film-forming ability, the texture of the object to be treated, the effect of improving the texture, and improving the durability of the effect, in particular, suppressing the entanglement of hair and improving the durability thereof when used for hair treatment.

The component (A) is preferably a polymer having a crosslinked structure. The term "crosslinked structure" as used herein refers to a three-dimensional network structure in which polymer chains, which are the main structure, are linked within a polymer chain or between polymer chains. The method for forming the crosslinked structure is not particularly limited, and examples thereof include a method of cross-linking simultaneously with polymerization such as polycondensation or radical polymerization, and a method of cross-linking polymer chains afterwards.

The component (A) may be used alone or in combination of two or more thereof. Among the above, the component (A) is preferably one or more selected from the group consisting of a methacryloyl ethyltrimethylammonium salt polymer and a quaternary dialkylaminoalkyl (meth)acrylic acid salt polymer, more preferably one or more selected from the group consisting of a methacryloyl ethyltrimethylammonium chloride polymer (polyquaternium-37), a methacryloyl ethyldimethylbetaine-methacryloyl trimethylammonium chloride-methacrylic acid methoxy polyethylene glycol copolymer (polyquaternium-49), a methacryloyl ethyl dimethyl betaine-methacryloyl ethyl trimethyl ammonium chloride-2-hydroxyethyl methacrylate copolymer (polyquaternium-48) and an N,N-dimethylaminoethyl methacrylic acid diethyl sulfate-N,N-dimethylacrylamide-polyethylene glycol dimethacrylate copolymer (polyquaternium-52), and even more preferably one or more selected from the group consisting of a methacryloyl ethyl trimethyl ammonium chloride polymer (polyquaternium-37) and an N,N-dimethylaminoethyl methacrylic acid diethyl sulfate-N,N-dimethylacrylamide/polyethylene glycol dimethacrylate copolymer (polyquaternium-52), from the viewpoint of easily setting the viscosity of a 1 mass% aqueous solution at 30°C to have a value equal to or larger than a predetermined value, from the viewpoint of the texture improving effect of the object to be treated and the improvement of the durability of the effect, particularly from the viewpoint of the effect of suppressing entanglement of hair and improving the durability thereof when used for hair treatment.

As the component (A), a commercially available polymer can also be used. Specific examples thereof include "Cosmedia Ultragel 300" (polyquaternium-37, cationic charge density: 4.81 mmol/g) manufactured by BASF Japan Ltd., "SOFCARE KG-101W-E" (polyquaternium-52, cationic charge density: 0.83 mmol/g), and "SOFCARE KG-301W" (polyquaternium-52, cationic charge density: 1.84 mmol/g) manufactured by Kao Corporation. All of these are cationic polymers having a crosslinked structure.

### (Component (B): Anionic Polymer)

The component (B) is a polymer capable of forming a polyion complex through interaction with the component (A). In the present specification, "anionic polymer" means a polymer having an anionic group and substantially having no cationic group and no amphoteric group. "Having substantially no cationic group and no amphoteric group" means that the molar amount of the cationic group and the amphoteric group relative to the anionic group is preferably 0.1% or less.

The anionic group of the component (B) is preferably an acidic group such as a carboxy group, a sulfonate group, or a phosphate group, and is more preferably a carboxy group from the viewpoint of facilitating formation of a polyion complex through interaction with the component (A) and from the viewpoint of availability. In the component (B), at least a part of the anionic groups may be neutralized to form a salt.

### <Component (B1): Crosslinked Polymer Containing (Meth)Acrylic Acid-Derived Constituent Unit, and Anionic Polysaccharides>

The component (B) preferably contains (B1) one or more selected from the group consisting of a crosslinked polymer containing a (meth)acrylic acid-derived constituent unit and an anionic polysaccharide, from the viewpoint of improving the effect of improving the texture of the object to be treated and the durability of the effect, particularly from the viewpoint of improving the effect of suppressing entanglement of hair and improving the durability thereof when used for hair treatment.

Among the components (B1), examples of the crosslinked polymer containing a constituent unit derived from (meth)acrylic acid include homopolymer or a copolymer of (meth)acrylic acid having a crosslinked structure, and a salt thereof. The term "crosslinked structure" as used herein refers to a three-dimensional network structure in which polymer chains, which are the main structure of a polymer, are linked within a polymer chain or between polymer chains. The method for forming the crosslinked structure is not particularly limited, and examples thereof include a method of cross-linking simultaneously with polymerization such as polycondensation or radical polymerization, and a method of cross-linking polymer chains afterwards.

Examples of the homopolymer of (meth)acrylic acid include a polyacrylic acid, a polymethacrylic acid, and a salt thereof.

Examples of the copolymer of (meth)acrylic acid include a (meth)acrylic acid/maleic acid copolymer, a (meth)acrylic acid/itaconic acid copolymer, a (meth)acrylic acid/fumaric acid copolymer, a (meth)acrylic acid/vinyl acetate copolymer, a (meth)acrylic acid/(meth)acrylic acid alkyl ester copolymer, a (meth)acrylic acid/2-hydroxyethyl methacrylate copolymer, an acrylic acid/acrylic acid alkyl ester/(N-alkyl)acrylamide copolymer, and a salt thereof, and one or two or more of these copolymers can be used.

Among these, as the copolymer of (meth)acrylic acid, a (meth)acrylic acid/(meth)acrylic acid alkyl ester is preferable, and an acrylic acid/acrylic acid alkyl ester is more preferable. Examples of the (meth)acrylic acid alkyl ester include (meth)acrylic acid alkyl esters in which the number of carbons of the alkyl is preferably 1 or more, more preferably 4 or more, and even more preferably 8 or more, and is preferably 40 or less, more preferably 36 or less, and even more preferably 32 or less.

Specific examples of the crosslinked polymer containing a constituent unit derived from (meth)acrylic acid include a carboxyvinyl polymer or a salt thereof, an (acrylates/alkyl acrylate (C10-30)) crosspolymer, a (Na acrylate/acryloyldimethyltaurine/dimethylacrylamide) crosspolymer, and an acrylates crosspolymer-4.

Among the components (B1), examples of the anionic polysaccharides include polysaccharides having a carboxy group (hyaluronic acid, alginic acid, pectic acid, carboxymethyl cellulose, xanthan gum, and the like), sulfates of polysaccharides (carrageenan, keratan sulfate, dermatan sulfate, sulfated starch, heparin, heparan sulfate), and a salt thereof, and one or two or more of these can be used.

Among the above, the anionic polysaccharide is preferably a polysaccharide having a carboxy group or a salt thereof, more preferably alginic acid, carboxymethyl cellulose, or a salt thereof, and even more preferably alginic acid or a salt thereof, from the viewpoint of improving the texture of the object to be treated and improving the durability of the effect, in particular, suppressing the entanglement of hair and improving the durability thereof when used for hair treatment.

When the component (B1) is a salt, examples of the salt include alkali metal salts such as a sodium salt and a potassium salt, and alkaline earth metal salts, and from the viewpoint of availability, an alkali metal salt is preferable.

Among the above, the component (B 1) is preferably one or more selected from the group consisting of a carboxyvinyl polymer, an (acrylates/alkyl acrylate (C10-30)) crosspolymer, and an anionic polysaccharide, more preferably an anionic polysaccharide or a salt thereof, and even more preferably alginic acid or a salt thereof, from the viewpoint of the texture improving effect of the object to be treated and the improvement of the durability of the effect, in particular, the effect of suppressing entanglement of hair and improving the durability thereof when used for hair treatment.

The anionic charge density of the component (B1) is not particularly limited, but is preferably 1.0 mmol/g or more, more preferably 2.0 mmol/g or more, and even more preferably 3.5 mmol/g or more from the viewpoint of facilitating formation of a polyion complex through interaction with the component (A), from the viewpoint of the texture improving effect of the object to be treated and the durability of the effect, particularly from the viewpoint of the effect of suppressing entanglement of hair and improving the durability thereof when used for hair treatment. The charge density is preferably 25 mmol/g or less, more preferably 20 mmol/g or less, and even more preferably 15 mmol/g or less, from the viewpoint of the effect of improving the texture of the object to be treated and the durability thereof, particularly from the viewpoint of the effect of suppressing entanglement of hair and improving the durability thereof when used for hair treatment. The anionic charge density of the component (B1) is preferably 1.0 mmol/g or more and 25 mmol/g or less, more preferably 2.0 mmol/g or more and 20 mmol/g or less, even more preferably 3.5 mmol/g or more and 15 mmol/g or less.

The anionic charge density of the component (B1) is the number of moles of anionic groups contained per polymer 1 g. When at least a part of the anion groups of the component (B1) is in the form of a neutralized salt, the number of moles of anion groups includes the number of moles of anion groups in the form of a salt.

Two or more polymers may be used as the component (B1), and in this case, the anionic charge density of the component (B1) is determined by calculating a weighted average from the anionic charge density and the blending amount of each polymer.

When the component (B1) is an anionic polysaccharide, the weight average molecular weight (Mw) thereof is preferably 2000 or more, more preferably 10000 or more, even more preferably more than 30000, and even more preferably 50000 or more from the viewpoint of improving the film-forming ability. The weight average molecular weight is preferably 3500000 or less, and more preferably 3000000 or less from the viewpoint of facilitating the formation of a polyion complex through the interaction with the component (A), the texture improving effect of the object to be treated and the improvement of the durability of the effect, particularly from the viewpoint of the effect of suppressing entanglement of hair and improving the durability thereof when used for hair treatment. The weight average molecular weight (Mw) of the anionic polysaccharide as the component (B1) is preferably 2000 or more and 3500000 or less, more preferably 10000 or more and 3000000 or less, even more preferably more than 30000 and 3000000 or less, and even more preferably 50000 or more and 3000000 or less.

The weight average molecular weight of the component (B1) can be measured by gel permeation chromatography (GPC).

The content of the component (B1) in the component (B) is preferably 50 mass% or more, more preferably 60 mass% or more, even more preferably 70 mass% or more, even more preferably 80 mass% or more, and is 100 mass% or less from the viewpoint of improving the film-forming ability, from the viewpoint of easily forming a polyion complex through interaction with the component (A), from the viewpoint of improving the texture improving effect of the object to be treated and the durability of the effect, particularly from the viewpoint of improving the entanglement suppressing effect of hair and improving the durability thereof when used for hair treatment.

### <Component (B2): Anionic Polymer Other Than Component (B1)>

The component (B) preferably further contains (B2) an anionic polymer other than the component (B1) from the viewpoint of further improving the texture improving effect of the object to be treated and the durability of the effect, particularly from the viewpoint of the effect of suppressing entanglement of hair and improving the durability thereof when used for hair treatment. It is considered that the component (B2) can immobilize the component (C) in the crosslinked structure of the polyion complex formed by the component (A)-component (B) by forming a hydrogen bond with the component (C) complexed with the crosslinked structure in the polyion complex. It is considered that due to this effect, even when rinsing or washing is performed after the object to be treated with the composition of the present invention is treated, the component (C) is not washed away and is likely to remain in the film, and the durability of the treatment effect is improved.

The molecular weight of the component (B2) is preferably 1000 or more, more preferably 2000 or more, even more preferably 5000 or more, even more preferably 10000 or more, and preferably 50000 or less, more preferably 40000 or less, even more preferably 30000 or less from the viewpoint of contributing to the immobilization of the component (C) to further improve the texture improving effect of the object to be treated and the durability of the effect, particularly from the viewpoint of improving the effect of suppressing entanglement of hair and improving the durability thereof when used for hair treatment. The molecular weight of the component (B2) is preferably 1000 or more and 50000 or less, more preferably 1000 or more and 30000 or less, even more preferably 2000 or more and 30000 or less, even more preferably 5000 or more and 30000 or less, and even more preferably 10000 or more and 30000 or less.

The term "molecular weight" as used herein means a weight average molecular weight and can be measured by gel permeation chromatography (GPC).

The component (B2) is preferably a polymer having a carboxy group with a molecular weight of 1000 or more and 50000 or less, more preferably 1000 or more and 30000 or less, and more preferably a non-crosslinked polymer containing a constituent unit derived from (meth)acrylic acid with a molecular weight of 1000 or more and 30000 or less.

Examples of the non-crosslinked polymer containing a constituent unit derived from (meth)acrylic acid suitably used as the component (B2) include, in addition to poly(meth)acrylic acid, a (meth)acrylic acid/maleic acid copolymer, a (meth)acrylic acid/itaconic acid copolymer, a (meth)acrylic acid/fumaric acid copolymer, a (meth)acrylic acid/vinyl acetate copolymer, a (meth)acrylic acid/(meth)acrylic acid alkyl ester copolymer, a (meth)acrylic acid/2-hydroxyethyl methacrylate copolymer, an acrylic acid/acrylic acid alkyl ester/(N-alkyl)acrylamide copolymer, and a salt thereof, and one or two or more of these can be used.

Examples of the polymer having a carboxy group other than those described above and suitably used as the component (B2) include polymaleic acid, a maleic acid/diisobutylene copolymer, a maleic acid/styrene copolymer, a benzoic acid formaldehyde condensate, a benzoic acid/phenol/formaldehyde condensate, and a salt thereof.

Among the above, the component (B2) is preferably one or more selected from the group consisting of a poly(meth)acrylic acid, a (meth)acrylic acid/(meth)acrylic acid alkyl ester copolymer, and a salt thereof from the viewpoint of further improving the texture improving effect of the object to be treated and the durability of the effect, particularly from the viewpoint of the effect of suppressing entanglement of hair and improving the durability thereof when used for hair treatment.

Examples of the poly(meth)acrylic acid or a salt thereof include polyacrylic acid, polymethacrylic acid, and a salt thereof, and polyacrylic acid or a salt thereof is preferable.

As the (meth)acrylic acid/(meth)acrylic acid alkyl ester copolymer or a salt thereof, an acrylic acid/acrylic acid alkyl ester or a salt thereof is more preferable. Examples of the (meth)acrylic acid alkyl ester include a (meth)acrylic acid alkyl ester in which the number of carbons in the alkyl is preferably 1 or more, more preferably 4 or more, even more preferably 8 or more, even more preferably 12 or more, and preferably 40 or less, more preferably 36 or less, even more preferably 32 or less, even more preferably 24 or less.

The (meth)acrylic acid/(meth)acrylic acid alkyl ester is preferably an acrylic acid/acrylic acid alkyl ester, more preferably one or more selected from the group consisting of an (acrylic acid/octyl acrylate) copolymer and an (acrylic acid/stearyl acrylate) copolymer, and even more preferably an (acrylic acid/stearyl acrylate) copolymer.

When the component (B2) is a salt, examples of the salt include alkali metal salts such as a sodium salt and a potassium salt, and alkaline earth metal salts, and from the viewpoint of availability, an alkali metal salt is preferable.

The anionic charge density of the component (B2) is not particularly limited, but is preferably 1.0 mmol/g or more, more preferably 3.0 mmol/g or more, and even more preferably 5.0 mmol/g or more, and is preferably 25 mmol/g or less, more preferably 20 mmol/g or less, and even more preferably 15 mmol/g or less from the viewpoint of further improving the texture improving effect of the object to be treated and the durability of the effect, particularly from the viewpoint of the effect of suppressing entanglement of hair and improving the durability thereof when used for hair treatment. The anionic charge density of the component (B2) is preferably 1.0 mmol/g or more and 25 mmol/g or less, more preferably 3.0 mmol/g or more and 20 mmol/g or less, even more preferably 5.0 mmol/g or more and 15 mmol/g or less.

Two or more polymers may be used as the component (B2), and in this case, the anionic charge density of the component (B2) is determined by calculating a weighted average from the anionic charge density and the blending amount of each polymer.

When the component (B) contains the component (B2), the content of the component (B2) in the component (B) is preferably 1 mass% or more, more preferably 2 mass% or more, even more preferably 3 mass% or more, and even more preferably 5 mass% or more, and is preferably 50 mass% or less, more preferably 40 mass% or less, even more preferably 30 mass% or less, and even more preferably 20 mass% or less from the viewpoint of further improving the texture improving effect of the object to be treated and the durability of the effect, particularly from the viewpoint of the effect of suppressing entanglement of hair and improving the durability thereof when used for hair treatment. The content of the component (B2) in the component (B) is preferably 1 mass% or more and 50 mass% or less, more preferably 2 mass% or more and 40 mass% or less, even more preferably 3 mass% or more and 30 mass% or less, and even more preferably 5 mass% or more and 20 mass% or less.

When the component (B) contains the component (B1) and the component (B2), the mass ratio of the component (B2) to the total amount of the component (B1) and the component (B2) [(B2)/{(B1) + (B2)}] is preferably 0.01 or more, more preferably 0.02 or more, even more preferably 0.05 or more, even more preferably 0.08 or more, and even more preferably 0.10 or more from the viewpoint of further improving the texture improving effect of the object to be treated and the durability of the effect, particularly from the viewpoint of suppressing the entanglement of hair and improving the durability thereof when used for hair treatment. From the viewpoint of easily forming a polyion complex with the component (A), the mass ratio is preferably 1.0 or less, more preferably 0.80 or less, even more preferably 0.50 or less, even more preferably 0.30 or less, and even more preferably 0.15 or less. The mass ratio [(B2)/{(B1) + (B2)}] is preferably 0.01 or more and 1.0 or less, more preferably 0.02 or more and 0.80 or less, even more preferably 0.05 or more and 0.50 or less, even more preferably 0.08 or more and 0.30 or less, even more preferably 0.10 or more, and even more preferably 0.15 or less.

### (Component (C))

The composition of the present invention contains one or more selected from the group consisting of (C1) a nonionic polymer and (C2) agar as the component (C). In the present specification, the "nonionic polymer" means a polymer having substantially no cationic group, anionic group, or amphoteric group, which are ionic groups.

### [Component (C1): Nonionic polymer]

Examples of the component (C1) include polyalkylene glycol or a derivative thereof, a polymer containing a constituent unit derived from vinylpyrrolidone, a polymer containing a constituent unit derived from a (meth)acrylic acid ester, polyvinyl alcohol, polyacrylamide, and polycaprolactam, and one or two or more thereof can be used.

Examples of the polyalkylene glycol include homopolymers and copolymers of alkylene glycols in which the number of carbons of alkylene is 2 or more and 6 or less, preferably 2 or more and 4 or less, and more preferably 2 or more and 3 or less. Specific examples thereof include polyethylene glycol, polypropylene glycol, polytrimethylene ether glycol, polytetramethylene ether glycol, a polyethylene glycol-polypropylene glycol copolymer (polyoxyethylene polyoxypropylene glycol), a polyethylene glycol-polytrimethylene ether glycol copolymer, a polyethylene glycol-polytetramethylene ether glycol copolymer, and a polypropylene glycol-polytetramethylene ether glycol copolymer. Among these, one or more selected from the group consisting of polyethylene glycol, polypropylene glycol, and a polyethylene glycol-polypropylene glycol copolymer is preferable, and one or more selected from the group consisting of polyethylene glycol and a polyethylene glycol-polypropylene glycol copolymer is more preferable, from the viewpoint of improving the effect of improving the texture of the object to be treated and the durability of the effect, in particular, improving the effect of suppressing entanglement of hair and improving the durability thereof when used for hair treatment, and from the viewpoint of availability.

Examples of the polyalkylene glycol derivative include terminal-modified products of the above-described polyalkylene glycols, such as polyalkylene glycol monoalkyl ethers, polyalkylene glycol dialkyl ethers, polyalkylene glycol monoaryl ethers, polyalkylene glycol diaryl ethers, polyalkylene glycol monofatty acid esters, polyalkylene glycol difatty acid esters, and polyalkylene glycol diglycidyl ethers. Among these, one or more selected from the group consisting of a polyalkylene glycol monoalkyl ether, a polyalkylene glycol dialkyl ether, a polyalkylene glycol monofatty acid ester, and a polyalkylene glycol difatty acid ester is preferable, and one or more selected from the group consisting of a polyalkylene glycol monofatty acid ester and a polyalkylene glycol difatty acid ester is more preferable, from the viewpoint of improving the texture improving effect of the object to be treated and the durability of the effect, in particular, suppressing the entanglement of hair and improving the durability thereof when used for hair treatment, and from the viewpoint of availability.

The number of carbons of the alkyl group in the mono- or dialkyl ether of polyalkylene glycol and the number of carbons of the fatty acid in the mono- or di-fatty acid ester of polyalkylene glycol are preferably 1 or more and 36 or less, more preferably 4 or more and 32 or less, even more preferably 8 or more and 24 or less, and even more preferably 12 or more and 22 or less. The number of carbons of the aryl group in the mono- or diaryl ether of polyalkylene glycol is preferably 6 or more and 36 or less, more preferably 6 or more and 32 or less, even more preferably 6 or more and 24 or less, and even more preferably 6 or more and 22 or less.

Specific examples of the polyalkylene glycol derivative include polyethylene glycol monomethyl ether, polyethylene glycol dimethyl ether, polyethylene glycol monobutyl ether, polyethylene glycol dibutyl ether, polyethylene glycol monooctyl ether, polyethylene glycol monodecyl ether, polyethylene glycol monododecyl ether, polyethylene glycol monotridecyl ether, polyethylene glycol-bisphenol A ether, polyethylene glycol dilaurate, polyethylene glycol distearate, polyethylene glycol diglycidyl ether, polyethylene glycol-polypropylene glycol-monomethyl ether, polyethylene glycol-polypropylene glycol-dimethyl ether, polyethylene glycol-polypropylene glycol-monobutyl ether, polyethylene glycol-polypropylene glycol-dibutyl ether, polyethylene glycol-polypropylene glycol-monooctyl ether, polyethylene glycol-polypropylene glycol-monodecyl ether, polyethylene glycol-polypropylene glycol-monododecyl ether, polyethylene glycol-polypropylene glycol-monotridecyl ether, polyethylene glycol-polypropylene glycol-bisphenol A ether, polyethylene glycol-polypropylene glycol-dilaurate, polyethylene glycol-polypropylene glycol distearate, and polyethylene glycol-polypropylene glycol-diglycidyl ether, and one or two or more of them can be used.

Examples of the polymer containing a constituent unit derived from vinylpyrrolidone include a homopolymer of vinylpyrrolidone and a copolymer of vinylpyrrolidone and a nonionic monomer. Specific examples thereof include polyvinylpyrrolidone, a vinylpyrrolidone/vinyl acetate copolymer, and a vinylpyrrolidone/methacrylamide/vinylimidazole copolymer.

Examples of the polymer containing a constituent unit derived from a (meth)acrylic acid ester include a homopolymer of a (meth)acrylic acid ester, and a copolymer of a (meth)acrylic acid ester and a nonionic monomer such as (meth)acrylamide, dimethylacrylamide, vinyl acetate, or vinyl methyl ether. Examples of the (meth)acrylic acid ester include (meth)acrylic acid hydroxyalkyl ester and (meth)acrylic acid alkyl ether in addition to the (meth)acrylic acid alkyl ester described above.

Specific examples of the polymer containing a constituent unit derived from a (meth)acrylic acid ester include a (dimethylacrylamide/hydroxyethyl acrylate/methoxyethyl acrylate) copolymer and a (hydroxyethyl acrylate/methoxyethyl acrylate) copolymer.

Among the above, the component (C1) is preferably one or more selected from the group consisting of polyalkylene glycol or a derivative thereof and a polymer containing a constituent unit derived from vinylpyrrolidone, more preferably one or more selected from the group consisting of polyethylene glycol, polypropylene glycol, a polyethylene glycol-polypropylene glycol copolymer, a mono- or di-fatty acid ester thereof, and polyvinylpyrrolidone, even more preferably one or more selected from the group consisting of polyethylene glycol, a polyethylene glycol-polypropylene glycol copolymer, a mono- or di-fatty acid ester thereof, and polyvinylpyrrolidone, and even more preferably one or more selected from the group consisting of polyethylene glycol, a polyethylene glycol-polypropylene glycol copolymer, polyethylene glycol distearate (polyethylene glycol distearate), and polyvinylpyrrolidone, from the viewpoint of the texture improving effect of the object to be treated and the improvement of the durability of the effect, particularly, the effect of suppressing entanglement of hair and improving the durability thereof when used for hair treatment, and from the viewpoint of the availability.

The weight average molecular weight (Mw) of the component (C1) is preferably 2000 or more, more preferably 5000 or more, even more preferably 10000 or more, and preferably 2000000 or less, more preferably 1500000 or less from the viewpoint of the texture improving effect of the object to be treated and the improvement of the durability of the effect, particularly the effect of suppressing entanglement of hair and improving the durability thereof when used for hair treatment, and the viewpoint of the availability. The weight average molecular weight of the component (C) is preferably 2000 or more and 2000000 or less, more preferably 5000 or more and 1500000 or less, and even more preferably 10000 or more and 1500000 or less.

The weight average molecular weight of the component (C1) can be measured by gel permeation chromatography (GPC).

### [Component (C2): Agar]

(C2) Agar is not particularly limited as long as it is formed of nonionic agarose as a main component and a small amount of anionic agaropectin, and commercially available products can be used.

The jelly strength of a 1.5% aqueous solution of the component (C2) is preferably 10 g or more, more preferably 50 g or more, even more preferably 100 g or more, even more preferably 200 g or more, and preferably 1500 g or less, more preferably 1000 g or less, more preferably 800 g or less, from the viewpoint of the texture improving effect of the object to be treated and the durability of the effect, in particular, the hair entanglement suppressing effect when used for hair treatment and improving the durability thereof. The jelly strength of a 1.5% aqueous solution of the component (C2) is preferably 10 g or more and 1500 g or less, more preferably 50 g or more and 1000 g or less, even more preferably 100 g or more and 800 g or less, and even more preferably 200 g or more and 800 g or less.

The jelly strength of the component (C2) is the maximum weight (g number) that can be withstood for 20 seconds per 1 cm² of the surface of a gel obtained by allowing a 1.5% aqueous solution to stand at 20°C for 15 hours and coagulating the gel, and can be determined by a Nikkansui-type measuring method.

Examples of commercially available products of the component (C2) include "S-5", "S-6", "S-7", "S-8", "S-9", "T-1", "T-2", "T-3", "T-88", "T-2000", "ZR", "Z-10", "ZL", "ZH", "M-7", "M-8", "M-9", "AX-30", "AX-100", "AX-200", "BX-30", "BX-100", "BX-200", "UP-16", "UP-26", "UP-37", "UX-30", "UX-100", "UX-200", "Yawara S", "Karikorikan", PS series, and CS series manufactured by Ina Food Industry Co., Ltd.

### (Water)

Since the composition of the present invention is an aqueous composition, it contains water. The water is preferably deionized water or distilled water. Tap water, ground water or the like sterilized with hypochlorous acid or the like may be used as long as the stability of the composition is not impaired.

### (Content)

The content or blending amount of each component in the aqueous composition is preferably as follows from the viewpoint of improving the texture of the object to be treated and improving the durability of the effect, particularly the effect of suppressing entanglement of hair and improving the durability thereof when used for hair treatment.

The content of the component (A) in the aqueous composition is preferably 0.01 mass% or more, more preferably 0.02 mass% or more, even more preferably 0.05 mass% or more, even more preferably 0.1 mass% or more, and even more preferably 0.2 mass% or more from the viewpoint of ease of formation of a polyion complex, the effect of improving the texture of an object to be treated and the durability of the effect, particularly the effect of suppressing entanglement of hair when used for hair treatment and the durability thereof. From the viewpoint of the stability of the aqueous composition, the content is preferably 3.0 mass% or less, more preferably 2.5 mass% or less, even more preferably 2.0 mass% or less, even more preferably 1.5 mass% or less, and even more preferably 1.0 mass% or less. The content of the component (A) in the aqueous composition is preferably 0.01 mass% or more and 3.0 mass% or less, more preferably 0.02 mass% or more and 2.5 mass% or less, even more preferably 0.05 mass% or more and 2.0 mass% or less, even more preferably 0.1 mass% or more and 1.5 mass% or less, even more preferably 0.2 mass% or more and 1.5 mass% or less, and even more preferably 0.2 mass% or more and 1.0 mass% or less.

The content of the component (B) in the aqueous composition is preferably 0.005 mass% or more, more preferably 0.01 mass% or more, even more preferably 0.02 mass% or more, and even more preferably 0.04 mass% or more from the viewpoint of ease of formation of a polyion complex, the effect of improving the texture of an object to be treated and the durability of the effect, particularly the effect of suppressing entanglement of hair when used for hair treatment and the durability thereof. From the viewpoint of the stability of the aqueous composition, the content is preferably 3.0 mass% or less, more preferably 2.0 mass% or less, even more preferably 1.5 mass% or less, even more preferably 1.0 mass% or less, even more preferably 0.5 mass% or less, even more preferably 0.3 mass% or less, even more preferably 0.2 mass% or less, and even more preferably 0.1 mass% or less. The content of the component (B) in the aqueous composition is preferably 0.005 mass% or more and 3.0 mass% or less, more preferably 0.01 mass% or more and 2.0 mass% or less, even more preferably 0.02 mass% or more and 1.5 mass% or less, even more preferably 0.02 mass% or more and 1.0 mass% or less, even more preferably 0.02 mass% or more and 0.5 mass% or less, even more preferably 0.02 mass% or more and 0.3 mass% or less, even more preferably 0.02 mass% or more and 0.3 mass% or less, even more preferably 0.02 mass% or more and 0.2 mass% or less, even more preferably 0.02 mass% or more and 0.1 mass% or less, and even more preferably 0.04 mass% or more and 0.1 mass% or less.

The total content of the component (A) and the component (B) in the aqueous composition is preferably 0.02 mass% or more, more preferably 0.04 mass% or more, even more preferably 0.05 mass% or more, even more preferably 0.1 mass% or more, even more preferably 0.2 mass% or more, and even more preferably 0.3 mass% or more from the viewpoint of ease of formation of a polyion complex, the effect of improving the texture of an object to be treated and the durability of the effect, particularly the effect of suppressing entanglement of hair when used for hair treatment and the durability thereof. From the viewpoint of the stability of the aqueous composition, the content is preferably 5.0 mass% or less, more preferably 4.0 mass% or less, even more preferably 3.0 mass% or less, and even more preferably 2.0 mass% or less. The total content of the component (A) and the component (B) in the aqueous composition is preferably 0.02 mass% or more and 5.0 mass% or less, more preferably 0.04 mass% or more and 4.0 mass% or less, even more preferably 0.05 mass% or more and 3.0 mass% or less, even more preferably 0.1 mass% or more and 2.0 mass% or less, even more preferably 0.2 mass% or more and 2.0 mass% or less, and even more preferably 0.3 mass% or more and 2.0 mass% or less.

The ratio between the contents of the component (A) and the component (B) in the aqueous composition is such that the mass ratio of the component (B) to the component (A) [(A)/(B)] is preferably 1.0 or more, more preferably 2.0 or more, even more preferably 3.0 or more, even more preferably 4.0 or more, and even more preferably 5.0 or more, and is preferably 30 or less, more preferably 20 or less, even more preferably 15 or less, even more preferably 12 or less, and even more preferably 10 or less from the viewpoint of the ease of formation of a polyion complex, the texture improving effect of the object to be treated and the improvement of the durability of the effect, in particular, the effect of suppressing entanglement of hair and improving the durability thereof when used for hair treatment. The mass ratio [(A)/(B)] is preferably 1.0 or more and 30 or less, more preferably 2.0 or more and 20 or less, even more preferably 3.0 or more and 15 or less, even more preferably 4.0 or more and 12 or less, and even more preferably 5.0 or more and 10 or less.

**In** the aqueous composition of the present invention, the mass ratio between the component (A) and the component (B) acts more dominantly than the cationic charge/anionic charge ratio of the component (A) and the component (B) for the effect of the present invention.

The ratio of the number of moles of the cationic charges of the component (A) to the number of moles of the anionic charges of the component (B) in the aqueous composition is preferably 10/90 to 95/5, more preferably 20/80 to 95/5, and even more preferably 25/75 to 90/10 from the viewpoint of the effect of improving the texture of the object to be treated and the durability of the effect, particularly the effect of suppressing entanglement of hair when used for hair treatment and the durability of the effect.

The content of the component (C) in the aqueous composition is preferably 0.005 mass% or more, more preferably 0.01 mass% or more, and even more preferably 0.02 mass% or more from the viewpoint of the effect of improving the texture of an object to be treated and the durability of the effect, particularly the effect of suppressing entanglement of hair when used for hair treatment and the durability thereof. From the viewpoint of the stability of the aqueous composition, the content is preferably 5.0 mass% or less, more preferably 4.0 mass% or less, even more preferably 3.0 mass% or less, and even more preferably 2.0 mass% or less. The content of the component (C) in the aqueous composition is preferably 0.005 mass% or more and 5.0 mass% or less, more preferably 0.01 mass% or more and 4.0 mass% or less, even more preferably 0.02 mass% or more and 3.0 mass% or less, and even more preferably 0.02 mass% or more and 2.0 mass% or less.

When the aqueous composition contains the component (C1), the content of the component (C1) in the aqueous composition is preferably 0.02 mass% or more, more preferably 0.04 mass% or more, even more preferably 0.05 mass% or more, even more preferably 0.1 mass% or more, even more preferably 0.2 mass% or more, and even more preferably 0.3 mass% or more from the viewpoint of ease of formation of a polyion complex, the effect of improving the texture of an object to be treated and the durability of the effect, particularly the effect of suppressing entanglement of hair when used for hair treatment and the durability thereof. From the viewpoint of the stability of the aqueous composition, the content is preferably 5.0 mass% or less, more preferably 4.0 mass% or less, even more preferably 3.0 mass% or less, and even more preferably 2.0 mass% or less. The content of the component (C1) in the aqueous composition is preferably 0.02 mass% or more and 5.0 mass% or less, more preferably 0.04 mass% or more and 4.0 mass% or less, even more preferably 0.05 mass% or more and 3.0 mass% or less, even more preferably 0.1 mass% or more and 2.0 mass% or less, even more preferably 0.2 mass% or more and 2.0 mass% or less, and even more preferably 0.3 mass% or more and 2.0 mass% or less.

When the aqueous composition contains the component (C2), the content of the component (C2) in the aqueous composition is preferably 0.005 mass% or more, more preferably 0.01 mass% or more, and even more preferably 0.02 mass% or more from the viewpoint of the effect of improving the texture of an object to be treated and the durability of the effect, particularly the effect of suppressing entanglement of hair when used for hair treatment and the durability thereof. From the viewpoint of the stability of the aqueous composition, the content is preferably 1.0 mass% or less, more preferably 0.5 mass% or less, even more preferably 0.2 mass% or less, and even more preferably 0.1 mass% or less. The content of the component (C2) in the aqueous composition is preferably 0.005 mass% or more and 1.0 mass% or less, more preferably 0.01 mass% or more and 0.5 mass% or less, even more preferably 0.01 mass% or more and 0.2 mass% or less, and even more preferably 0.02 mass% or more and 0.1 mass% or less.

The mass ratio of the component (C) to the total amount of the component (A) and the component (B) in the aqueous composition [(C)/{(A) + (B)}] is preferably 0.01 or more, more preferably 0.02 or more, and even more preferably 0.03 or more, and is preferably 20 or less, more preferably 15 or less, even more preferably 10 or less, even more preferably 8.0 or less, even more preferably 5.0 or less, even more preferably 2.0 or less, and even more preferably 1.5 or less from the viewpoint of the ease of formation of a polyion complex, the texture improving effect of the object to be treated and the improvement of the durability of the effect, in particular, the effect of suppressing entanglement of hair and improving the durability thereof when used for hair treatment. The mass ratio [(C)/{(A) + (B)}] is preferably 0.01 or more and 20 or less, more preferably 0.02 or more and 15 or less, even more preferably 0.02 or more and 10 or less, even more preferably 0.02 or more and 8.0 or less, even more preferably 0.02 or more and 5.0 or less, even more preferably 0.02 or more and 2.0 or less, and even more preferably 0.03 or more and 1.5 or less.

When the aqueous composition contains the composition (C1), the mass ratio of the component (C1) to the total amount of the component (A) and the component (B) [(C1)/{(A) + (B)}] in the aqueous composition is preferably 0.2 or more, more preferably 0.3 or more, even more preferably 0.4 or more, even more preferably 0.5 or more, even more preferably 0.6 or more, even more preferably 0.8 or more, and preferably 20 or less, more preferably 15 or less, even more preferably 10 or less, even more preferably 8.0 or less, even more preferably 5.0 or less, even more preferably 2.0 or less, even more preferably 1.5 or less from the viewpoint of an effect of improving the texture of the object to be treated and the durability of the effect, particularly from the viewpoint of an effect of suppressing entanglement of hair and improving the durability thereof when used for hair treatment. The mass ratio [(C1)/{(A) + (B)}] is preferably 0.2 or more and 20 or less, more preferably 0.3 or more and 15 or less, even more preferably 0.4 or more and 10 or less, even more preferably 0.5 or more and 8.0 or less, even more preferably 0.6 or more and 5.0 or less, even more preferably 0.8 or more and 2.0 or less, and even more preferably 0.8 or more and 1.5 or less.

When the aqueous composition contains the component (C2), the mass ratio of the component (C2) to the total amount of the component (A) and the component (B) in the aqueous composition [(C2)/{ (A) + (B)}] is preferably 0.01 or more, more preferably 0.02 or more, and even more preferably 0.03 or more, and is preferably 1.0 or less, more preferably 0.5 or less, even more preferably 0.3 or less, and even more preferably 0.2 or less from the viewpoint of the ease of formation of a polyion complex, the texture improving effect of the object to be treated and the improvement of the durability of the effect, in particular, the effect of suppressing entanglement of hair and improving the durability thereof when used for hair treatment. The mass ratio [(C2)/{(A) + (B)}] is preferably 0.01 or more and 1.0 or less, more preferably 0.02 or more and 0.5 or less, even more preferably 0.02 or more and 0.3 or less, and even more preferably 0.03 or more and 0.2 or less.

The content of water in the aqueous composition is preferably 50 mass% or more, more preferably 70 mass% or more, even more preferably 80 mass% or more, and even more preferably 90 mass% or more from the viewpoint of obtaining an aqueous composition and the stability of the composition. The content of water in the aqueous composition may be the balance of the components (A) to (C).

In addition, the composition of the present invention may contain, as optional components, preservatives, pH adjusters (acids, bases, or salts thereof), salt compounds (sodium chloride and the like), aqueous media other than water, and the like.

### (pH)

From the viewpoint of safety when used for body, the pH of the aqueous composition is preferably 3.0 or more, more preferably 3.2 or more as the pH at 25°C of an aqueous solution obtained by diluting the aqueous composition with water by 20 times. The pH is preferably 7.5 or less, more preferably 7.0 or less, even more preferably 6.0 or less, and even more preferably 5.0 or less from the viewpoint of the texture improving effect of the object to be treated and the improvement of the durability of the effect, particularly the effect of suppressing entanglement of hair when used for hair treatment and the improvement of the durability of the effect, and from the viewpoint of the stability of the aqueous composition.

The pH can be measured by the method described in Examples using a pH meter.

### (Method for Producing Aqueous Composition)

The method for producing the composition of the present invention is not particularly limited, but the following methods are preferable from the viewpoint of the stability of the aqueous composition, the texture improving effect of the object to be treated and the improvement of the durability of the effect, particularly from the viewpoint of the effect of suppressing entanglement of hair when used for hair treatment and the improvement of the durability of the effect, from the viewpoint of the stability of the aqueous composition, and from the viewpoint of the production efficiency.

When the component (C1) is used as the component (C), the method preferably includes a step of dissolving or dispersing the component (B) and the component (C1) in water to prepare a liquid mixture containing the component (B) and the component (C1), and then mixing the liquid mixture with the component (A) or an aqueous solution thereof. Through the method, the component (C1) is retained in the crosslinked structure formed by ionic interaction between the component (A) and the component (B), and a network structure can be efficiently formed.

When a pH adjusting agent is blended, it is preferable that a pH adjusting agent is added to the liquid mixture containing the component (B) and the component (C1) to adjust the pH to a desired pH, and then the liquid mixture is mixed with the component (A) or an aqueous solution thereof.

The mixing conditions for mixing the components (A) to (C1) are not particularly limited, and the mixing can be usually performed at from 5 to 60°C for from 0.1 to 12 hours using a known stirring device.

When the component (C2) is used as the component (C), the component (C) is preferably produced by a method including a step of preparing each of an aqueous solution of the component (A), an aqueous solution of the component (B), and an aqueous solution of the component (C2), and then mixing these aqueous solutions. When the component (B 1) and the component (B2) are used in combination as the component (B), it is preferable to prepare each of the aqueous solution of the component (B 1) and the aqueous solution of the component (B2) and to mix them with the aqueous solution of the component (C2).

When the aqueous composition does not contain (C1) a nonionic polymer but contains only (C2) agar as the component (C), the method for producing the composition of the present invention preferably includes a step of mixing the aqueous solution of the component (A) and the aqueous solution of the component (C2) under heating conditions of preferably 50°C or more and 100°C or less and more preferably 60°C or more and 95°C or less to prepare a liquid mixture, and then mixing the liquid mixture and the aqueous solution of the component (B). It is considered that a network having higher strength and including the component (A), the component (B), and the component (C2) is efficiently formed through this method.

When a pH adjusting agent such as an acid is blended, it is preferable that a pH adjusting agent is added to a liquid mixture obtained by mixing the aqueous solution of the component (A) and the aqueous solution of the component (C2) to adjust the pH to a desired pH, and then the liquid mixture is mixed with the aqueous solution of the component (B).

### [Cosmetic Composition]

The present invention also provides a cosmetic composition including the aqueous composition for body. By applying the cosmetic composition of the present invention to an object to be treated, the texture improving effect of the object to be treated and the durability of the effect can be improved. In particular, when the cosmetic composition of the present invention is used for hair treatment, the effect of suppressing entanglement of hair and the durability thereof can be improved.

Examples of the type of the cosmetic composition include a skin cosmetic composition and a hair cosmetic composition, and from the viewpoint of improving the effect of suppressing entanglement of hair and the durability thereof, the cosmetic composition is preferably a hair cosmetic composition.

The aqueous composition for body may be used as a cosmetic composition as it is. It is also possible to use the aqueous composition for body as a premix of a cosmetic composition. For example, various cosmetic compositions can be prepared by adding additional components to the aqueous composition.

Since the components (A) to (C) are stably present in a state of forming the network described above in the aqueous composition, the network is unlikely to be broken even when an additional component is blended. Thus, it is considered that the cosmetic composition containing the aqueous composition can maintain the effect of improving the texture of the object to be treated and the effect of improving the durability of the effect.

The content of the aqueous composition for body in the cosmetic composition is usually 1 mass% or more and 80 mass% or less, preferably 10 mass% or more and 60 mass% or less. In addition, the content of components (A) to (C) in the cosmetic composition may be preferably in the following range.

The content of the component (A) in the cosmetic composition is preferably 0.001 mass% or more, more preferably 0.005 mass% or more, even more preferably 0.01 mass% or more, and even more preferably 0.05 mass% or more, from the viewpoint of improving the texture improving effect of the object to be treated and the durability of the effect, particularly from the viewpoint of improving the effect of suppressing entanglement of hair and improving the durability thereof when used for hair treatment. The content is preferably 3.0 mass% or less, more preferably 2.0 mass% or less, even more preferably 1.5 mass% or less, and even more preferably 1.0 mass% or less from the viewpoint of stability of the composition and from the viewpoint of exhibiting the effect when additional components are blended. The content of the component (A) in the cosmetic composition is preferably 0.001 mass% or more and 3.0 mass% or less, more preferably 0.005 mass% or more and 2.0 mass% or less, even more preferably 0.01 mass% or more and 1.5 mass% or less, even more preferably 0.05 mass% or more and 1.5 mass% or less, and even more preferably 0.05 mass% or more and 1.0 mass% or less.

The content of the component (B) in the cosmetic composition is preferably 0.0005 mass% or more, more preferably 0.001 mass% or more, even more preferably 0.01 mass% or more, and even more preferably 0.02 mass% or more from the viewpoint of improving the texture improving effect of the object to be treated and the durability of the effect, particularly from the viewpoint of improving the effect of suppressing entanglement of hair and improving the durability thereof when used for hair treatment. **In** addition, from the viewpoint of stability of the composition and the viewpoint of exhibiting the effect when additional components are blended, the content is preferably 3.0 mass% or less, more preferably 2.0 mass% or less, even more preferably 1.5 mass% or less, even more preferably 1.0 mass% or less, even more preferably 0.5 mass% or less, even more preferably 0.3 mass% or less, even more preferably 0.2 mass% or less, and even more preferably 0.1 mass% or less. The content of the component (B) in the cosmetic composition is preferably 0.0005 mass% or more and 3.0 mass% or less, more preferably 0.001 mass% or more and 2.0 mass% or less, even more preferably 0.01 mass% or more and 1.5 mass% or less, even more preferably 0.02 mass% or more and 1.0 mass% or less, even more preferably 0.02 mass% or more and 0.5 mass% or less, even more preferably 0.02 mass% or more and 0.3 mass% or less, even more preferably 0.02 mass% or more and 0.2 mass% or less, and even more preferably 0.02 mass% or more and 0.1 mass% or less.

The total content of the component (A) and the component (B) in the cosmetic composition is preferably 0.0015 mass% or more, more preferably 0.01 mass% or more, even more preferably 0.02 mass% or more, and even more preferably 0.05 mass% or more from the viewpoint of improving the texture improving effect of the object to be treated and the durability of the effect, particularly from the viewpoint of improving the effect of suppressing entanglement of hair and improving the durability thereof when used for hair treatment. The content is preferably 5.0 mass% or less, more preferably 4.0 mass% or less, even more preferably 2.0 mass% or less, and even more preferably 1.5 mass% or less from the viewpoint of stability of the composition and from the viewpoint of exhibiting the effect when additional components are blended. The total content of the component (A) and the component (B) in the cosmetic composition is preferably 0.0015 mass% or more and 5.0 mass% or less, more preferably 0.01 mass% or more and 4.0 mass% or less, even more preferably 0.02 mass% or more and 4.0 mass% or less, even more preferably 0.05 mass% or more and 2.0 mass% or less, and even more preferably 0.05 mass% or more and 1.5 mass% or less.

The content of the component (C) in the cosmetic composition is preferably 0.001 mass% or more, more preferably 0.002 mass% or more, even more preferably 0.005 mass% or more, and even more preferably 0.01 mass% or more from the viewpoint of improving the texture improving effect of the object to be treated and the durability of the effect, particularly from the viewpoint of improving the effect of suppressing entanglement of hair and improving the durability thereof when used for hair treatment. The content is preferably 5.0 mass% or less, more preferably 4.0 mass% or less, even more preferably 2.0 mass% or less, and even more preferably 1.5 mass% or less from the viewpoint of stability of the composition and from the viewpoint of exhibiting the effect when additional components are blended. The content of the component (C) in the cosmetic composition is preferably 0.001 mass% or more and 5.0 mass% or less, more preferably 0.002 mass% or more and 4.0 mass% or less, even more preferably 0.005 mass% or more and 4.0 mass% or less, even more preferably 0.005 mass% or more and 2.0 mass% or less, and even more preferably 0.01 mass% or more and 1.5 mass% or less.

Examples of the additional component to be added to the cosmetic composition include components usually added to a skin cosmetic composition or a hair cosmetic composition, for example, surfactants, higher alcohols, oils, organic or inorganic acids, aqueous media, pH adjusting agents, texture improvers, coloring agents, antioxidants, anti-dandruff agents, vitamins, bactericides, anti-inflammatory agents, preservatives, chelating agents, humectants, pearl agents, ceramides, fragrances, plant extracts, and ultraviolet absorbers.

When the cosmetic composition contains an oily component such as a higher alcohol or an oil agent, the cosmetic composition may be in the form of an emulsion composition. From the viewpoint of the stability of the composition and the viewpoint of improving the use feeling, the emulsified composition is preferably an oil-in-water emulsion composition.

The method for producing the cosmetic composition in the form of an emulsion composition is not particularly limited. Examples thereof include a method of mixing the aqueous composition and an oily phase containing an oily component, and then adding and mixing an aqueous phase containing an aqueous component other than the aqueous composition and/or another oily component as necessary; and a method of mixing an oily phase containing an oily component and an aqueous phase containing an aqueous component other than the aqueous composition, and then adding and mixing the aqueous composition. From the viewpoint of producing a highly stable emulsion composition, improving the texture of the object to be treated and improving the durability of the effect, in particular, suppressing the entanglement of hair when used for hair treatment and improving the durability thereof, it is preferable to produce an emulsion composition by a method of mixing the aqueous composition with an oily phase containing an oily component, and then adding and mixing an aqueous phase containing an aqueous component other than the aqueous composition and/or another oily component as necessary.

The term "aqueous phase" means a phase constituting an aqueous phase in the production process of the emulsion composition, and "oily phase" means a phase constituting an oil phase in the production process of the emulsion composition.

Examples of the oily component contained in the oily phase referred to herein preferably include an ionic surfactant and an amphipathic substance having a hydrophobic group. Examples of the ionic surfactant include an anionic surfactant, a cationic surfactant, and an amphoteric surfactant, and examples of the amphipathic substance having a hydrophobic group include a higher alcohol. Examples of the "aqueous component other than the aqueous composition" include an organic or inorganic acid and an aqueous medium, and examples of the "another oily component" include an oil agent. Further, other components exemplified as "additional components" can be appropriately used for the emulsion composition.

Among the cosmetic compositions, examples of the product form of the skin cosmetic composition include a body soap, a facial cleanser, a bath agent, a deodorant preparation, a makeup cosmetic, a sunscreen, a makeup foundation, a milky lotion, a beauty essence, and a cream.

Examples of the product form of the hair cosmetic composition include a hair shampoo, a hair rinse, a hair conditioner, a hair treatment (including a leave-on type), a hair styling agent, a hair coloring agent, and a permanent agent. Among these, from the viewpoint of the effectiveness of the effects of the present invention, a hair conditioner, a hair treatment, or a hair styling agent is preferable.

### [Hair Treatment Method]

The present invention further provides a hair treatment method including a step of applying the cosmetic composition to hair and then heating the hair to a temperature of 80°C or more. According to the hair treatment method of the present invention, by applying the cosmetic composition to hair and then heating the hair, the hydrogen bond between the component (B) and the component (C) in the film formed on the hair surface is further strengthened, and thus the durability of the effect of suppressing entanglement of the hair can be further improved.

**In** the hair treatment method of the present invention, first, the cosmetic composition is applied to hair. Examples of the method for applying the cosmetic composition include a method of coating hair with the cosmetic composition, flowing, or spraying the cosmetic composition to hair, a method of immersing hair in the cosmetic composition, and the like, and preferably, a method of coating hair with the cosmetic composition.

As a specific example of a method for applying the cosmetic composition to hair, when the cosmetic composition is a hair shampoo, the hair shampoo is applied to the hair, and a step of foaming on the hair and then rinsing off is performed. When the cosmetic composition is a hair rinse, a hair conditioner, a hair treatment, or a hair pack, for example, a step of applying and spreading the cosmetic composition to washed hair and then rinsing off the composition as necessary is performed.

The hair to which the cosmetic composition is applied may be in either a dry state or a wet state, but is preferably in a wet state.

The application amount of the cosmetic composition to the hair is preferably 0.005 or more, more preferably 0.01 or more, even more preferably 0.05 or more, and preferably 20 or less, more preferably 15 or less, even more preferably 12 or less in terms of a bath ratio (mass of cosmetic composition/dry mass of hair to be applied) to the mass of the hair to be applied.

The hair to which the cosmetic composition is applied may be the entire hair or a part thereof.

It is preferable to perform a step of washing off the cosmetic composition with water after applying the cosmetic composition to hair and before heating the hair. In this case, to allow the cosmetic composition to be sufficiently applied to hair, a standing time may be provided after application of the cosmetic composition until the cosmetic composition is washed away with water. The standing time is preferably 15 seconds or more, more preferably 30 seconds or more, and even more preferably 1 minute or more, and is preferably 20 minutes or less, and even more preferably 15 minutes or less.

The time for washing off the cosmetic composition with water is preferably 5 seconds or more and 3 minutes or less. The temperature of the water may be such that it does not impose a burden on the body, and is preferably 15°C or more and 50°C or less, more preferably 25°C or more and 45°C or less.

The hair after application of the cosmetic composition is preferably subjected to heating after drying with a towel dryer, a dryer, or the like.

Preferable examples of the method for heating hair include a method using a flat iron. The heating temperature of hair is 80°C or more, preferably 100°C or more, more preferably 120°C or more, and even more preferably 140°C or more, from the viewpoint of further improving the durability of the effect of suppressing entanglement of hair. From the viewpoint of suppressing hair damage, the temperature is preferably 220°C or less, and more preferably 200°C or less.

The hair treated by the above method tends to maintain the effect that the hair is hardly entangled even when the hair is repeatedly washed.

With respect to the above-described embodiments, the present invention discloses the following.
<1> An aqueous composition for body, the aqueous composition containing (A) a cationic polymer having a viscosity when in a 1 mass% aqueous solution form at 30°C of 1000 mPa·s or more, (B) an anionic polymer, (C) one or more selected from the group consisting of (C1) a nonionic polymer and (C2) agar, and water.
<2> The aqueous composition for body according to <1>, wherein the viscosity of a 1 mass% aqueous solution of the component (A) at 30°C is preferably 1000 mPa·s or more and 100000 mPa·s or less, more preferably 1300 mPa·s or more and 80000 mPa·s or less, even more preferably 1500 mPa·s or more and 50000 mPa·s or less, even more preferably 1700 mPa·s or more and 30000 mPa·s or less, and even more preferably 1800 mPa·s or more and 25000 mPa·s or less.
<3> The aqueous composition for body according to <1> or <2>, wherein the component (A) has a cationic charge density of preferably 0.1 mmol/g or more and 20 mmol/g or less, more preferably 0.2 mmol/g or more and 15 mmol/g or less, 0.2 mmol/g or more and 12 mmol/g or less, even more preferably 0.3 mmol/g or more and 10 mmol/g or less, even more preferably 0.3 mmol/g or more and 7.0 mmol/g or less, and even more preferably 0.5 mmol/g or more and 5.0 mmol/g or less.
<4> The aqueous composition for body according to any one of <1> to <3>, wherein the component (A) is one or more selected from the group consisting of a cationized polyvinyl alcohol, a polyethyleneimine, a methacryloylethyltrimethylammonium salt polymer, a quaternized dialkylaminoalkyl (meth)acrylic acid salt polymer, a diallyl quaternized ammonium salt polymer, a methacrylamidopropyltrimethylammonium salt polymer, a vinylimidazolium trichloride-vinylpyrrolidone copolymer (polyquaternium-16), a vinylpyrrolidone-alkylamino (meth)acrylate copolymer, a vinylpyrrolidone-alkylamino (meth)acrylate-vinylcaprolactam copolymer, an alkylacrylamide-(meth)acrylate-alkylaminoalkylacrylamide-polyethylene glycol (meth)acrylate copolymer, and an adipic acid-dimethylaminohydroxypropylethylenetriamine copolymer and has a viscosity when in a 1 mass% aqueous solution form at 30°C of 1000 mPa·s or more.
<5> The aqueous composition for body according to any one of <1> to <4>, wherein the component (A) is a polymer containing a constituent unit represented by General Formula (1) described below: 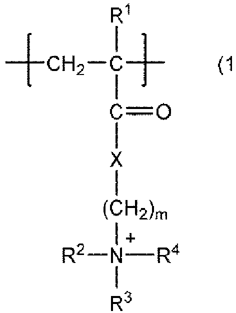 wherein R¹ is a hydrogen atom or a methyl group, R² to R⁴ are each independently an alkyl group having 1 or more and 4 or less carbons, X is -O- or -NH-, and m is a number of 1 to 4.
<6> The aqueous composition for body according to <5>, wherein the constituent unit represented by General Formula (1) is one or more selected from the group consisting of a constituent unit derived from a methacryloylethyltrimethylammonium salt, a constituent unit derived from N,N-dimethylaminoethylmethacrylic acid diethyl sulfate, and a constituent unit derived from a methacrylamidopropyltrimethylammonium salt, and preferably one or more selected from the group consisting of a constituent unit derived from a methacryloylethyltrimethylammonium salt and a constituent unit derived from N,N-dimethylaminoethylmethacrylic acid diethyl sulfate.
<7> The aqueous composition for body according to <5> or <6>, wherein a content of the constituent unit represented by General Formula (1) in the component (A) is 8.0 mol% or more and 100 mol% or less.
<8> The aqueous composition for body according to any one of <1> to <7>, wherein the component (A) is preferably one or more selected from the group consisting of a methacryloylethyltrimethylammonium salt polymer and a quaternized dialkylaminoalkyl (meth)acrylic acid salt polymer, more preferably one or more selected from the group consisting of a methacryloylethyltrimethylammonium chloride polymer (polyquaternium-37), a methacryloylethyldimethylbetaine-methacryloylethyltrimethylammonium chloride-methoxypolyethylene glycol methacrylate copolymer (polyquaternium-49), a methacryloylethyldimethylbetaine-methacryloylethyltrimethylammonium chloride-2-hydroxyethyl methacrylate copolymer (polyquaternium-48), and an N,N-dimethylaminoethylmethacrylic acid diethyl sulfate-N,N-dimethylacrylamide-polyethylene glycol dimethacrylate copolymer (polyquaternium-52), even more preferably one or more selected from the group consisting of a methacryloylethyltrimethylammonium chloride polymer (polyquaternium-37) and an N,N-dimethylaminoethylmethacrylic acid diethyl sulfate-N,N-dimethylacrylamide-polyethylene glycol dimethacrylate copolymer (polyquaternium-52).
<9> The aqueous composition for body according to any one of <1> to <8>, wherein the component (B) contains (B1) one or more selected from the group consisting of a crosslinked polymer containing a constituent unit derived from (meth)acrylic acid and an anionic polysaccharide.
<10> The aqueous composition for body according to <9>, wherein the crosslinked polymer containing a constituent unit derived from (meth)acrylic acid is a homopolymer or copolymer of (meth)acrylic acid having a crosslinked structure or a salt thereof, preferably one or more selected from the group consisting of a carboxyvinyl polymer or a salt thereof, an (acrylates/alkyl acrylate (C10-30)) crosspolymer, a (Na acrylate/acryloyldimethyltaurine/dimethylacrylamide) crosspolymer, and an acrylates crosspolymer-4.
<11> The aqueous composition for body according to <9> or <10>, wherein the anionic polysaccharide is one or more selected from the group consisting of a polysaccharide having a carboxy group, a sulfate of a polysaccharide, and a salt thereof.
<12> The aqueous composition for body according to any one of <1> to <11>, wherein the component (B 1) is preferably one or more selected from the group consisting of a carboxyvinyl polymer, an (acrylates/alkyl acrylate (C10-30)) crosspolymer, and an anionic polysaccharide, more preferably an anionic polysaccharide or a salt thereof, and even more preferably alginic acid or a salt thereof.
<13> The aqueous composition for body according to any one of <9> to <12>, wherein the component (B1) has an anionic charge density of preferably 1.0 mmol/g or more and 25 mmol/g or less, more preferably 2.0 mmol/g or more and 20 mmol/g or less, even more preferably 3.5 mmol/g or more and 15 mmol/g or less.
<14> The aqueous composition for body according to any one of <9> to <13>, wherein the anionic polysaccharide has a weight average molecular weight (Mw) of preferably 2000 or more and 3500000 or less, more preferably 10000 or more and 3000000 or less, even more preferably more than 30000 and 3000000 or less, and even more preferably 50000 or more and 3000000 or less.
<15> The aqueous composition for body according to any one of <9> to <14>, wherein a content of the component (B1) in the component (B) is preferably 50 mass% or more, more preferably 60 mass% or more, even more preferably 70 mass% or more, even more preferably 80 mass% or more, and is 100 mass% or less.
<16> The aqueous composition for body according to any one of <9> to <15>, wherein the component (B) further contains (B2) an anionic polymer other than the component (B1).
<17> The aqueous composition for body according to <16>, wherein the component (B2) has a molecular weight of preferably 1000 or more and 50000 or less, more preferably 1000 or more and 30000 or less, even more preferably 2000 or more and 30000 or less, even more preferably 5000 or more and 30000 or less, and even more preferably 10000 or more and 30000 or less.
<18> The aqueous composition for body according to <16> or <17>, wherein the component (B2) is preferably a polymer having a carboxy group with a molecular weight of 1000 or more and 50000 or less, more preferably 1000 or more and 30000 or less, and more preferably a non-crosslinked polymer containing a constituent unit derived from (meth)acrylic acid with a molecular weight of 1000 or more and 30000 or less.
<19> The aqueous composition for body according to any one of <16> to <18>, wherein the component (B2) is one or more selected from the group consisting of a poly(meth)acrylic acid, a (meth)acrylic acid/(meth)acrylic acid alkyl ester copolymer, and a salt thereof.
<20> The aqueous composition for body according to any one of <16> to <19>, wherein the component (B2) has an anionic charge density of preferably 1.0 mmol/g or more and 25 mmol/g or less, more preferably 3.0 mmol/g or more and 20 mmol/g or less, even more preferably 5.0 mmol/g or more and 15 mmol/g or less.
<21> The aqueous composition for body according to any one of <16> to <20>, wherein a content of the component (B2) in the component (B) is preferably 1 mass% or more and 50 mass% or less, more preferably 2 mass% or more and 40 mass% or less, even more preferably 3 mass% or more and 30 mass% or less, even more preferably 5 mass% or more and 20 mass% or less.
<22> The aqueous composition for body according to any one of <16> to <21>, wherein a mass ratio [(B2)/{(B 1) + (B2)}] of the component (B2) to a total amount of the component (B1) and the component (B2) is preferably 0.01 or more and 1.0 or less, more preferably 0.02 or more and 0.80 or less, even more preferably 0.05 or more and 0.50 or less, even more preferably 0.08 or more and 0.30 or less, even more preferably 0.10 or more, and even more preferably 0.15 or less.
<23> The aqueous composition for body according to any one of <1> to <22>, wherein the component (C1) is one or more selected from the group consisting of a polyalkylene glycol or a derivative thereof, a polymer containing a constituent unit derived from vinylpyrrolidone, a polymer containing a constituent unit derived from a (meth)acrylic acid ester, polyvinyl alcohol, polyacrylamide, and polycaprolactam, preferably one or more selected from the group consisting of a polyalkylene glycol or a derivative thereof and a polymer containing a constituent unit derived from vinylpyrrolidone, more preferably one or more selected from the group consisting of polyethylene glycol, polypropylene glycol, a polyethylene glycol-polypropylene glycol copolymer, a mono- or di-fatty acid ester thereof, and polyvinylpyrrolidone, even more preferably one or more selected from the group consisting of polyethylene glycol, a polyethylene glycol-polypropylene glycol copolymer, a mono- or di-fatty acid ester thereof, and polyvinylpyrrolidone, and even more preferably one or more selected from the group consisting of polyethylene glycol, a polyethylene glycol-polypropylene glycol copolymer, polyethylene glycol distearate (polyethylene glycol distearate), and polyvinylpyrrolidone.
<24> The aqueous composition for body according to any one of <1> to <23>, wherein the component (C1) has a weight average molecular weight of preferably 2000 or more and 2000000 or less, more preferably 5000 or more and 1500000 or less, and even more preferably 10000 or more and 1500000 or less.
<25> The aqueous composition for body according to any one of <1> to <24>, wherein a 1.5% aqueous solution of the component (C2) has a jelly strength of preferably 10 g or more and 1500 g or less, more preferably 50 g or more and 1000 g or less, even more preferably 100 g or more and 800 g or less, and even more preferably 200 g or more and 800 g or less.
<26> The aqueous composition for body according to any one of <1> to <25>, wherein a content of the component (A) in the aqueous composition for body is preferably 0.01 mass% or more and 3.0 mass% or less, more preferably 0.02 mass% or more and 2.5 mass% or less, even more preferably 0.05 mass% or more and 2.0 mass% or less, even more preferably 0.1 mass% or more and 1.5 mass% or less, even more preferably 0.2 mass% or more and 1.5 mass% or less, and even more preferably 0.2 mass% or more and 1.0 mass% or less.
<27> The aqueous composition for body according to any one of <1> to <26>, wherein a content of the component (B) in the aqueous composition for body is preferably 0.005 mass% or more and 3.0 mass% or less, more preferably 0.01 mass% or more and 2.0 mass% or less, even more preferably 0.02 mass% or more and 1.5 mass% or less, even more preferably 0.02 mass% or more and 1.0 mass% or less, even more preferably 0.02 mass% or more and 0.5 mass% or less, even more preferably 0.02 mass% or more and 0.3 mass% or less, even more preferably 0.02 mass% or more and 0.3 mass% or less, even more preferably 0.02 mass% or more and 0.2 mass% or less, even more preferably 0.02 mass% or more and 0.1 mass% or less, and even more preferably 0.04 mass% or more and 0.1 mass% or less.
<28> The aqueous composition for body according to any one of <1> to <27>, wherein a total content of the component (A) and the component (B) in the aqueous composition for body is preferably 0.02 mass% or more and 5.0 mass% or less, more preferably 0.04 mass% or more and 4.0 mass% or less, even more preferably 0.05 mass% or more and 3.0 mass% or less, even more preferably 0.1 mass% or more and 2.0 mass% or less, even more preferably 0.2 mass% or more and 2.0 mass% or less, and even more preferably 0.3 mass% or more and 2.0 mass% or less.
<29> The aqueous composition for body according to any one of <1> to <28>, wherein a mass ratio [(A)/(B)] of the component (A) to the component (B) in the aqueous composition for body is preferably 1.0 or more and 30 or less, more preferably 2.0 or more and 20 or less, even more preferably 3.0 or more and 15 or less, even more preferably 4.0 or more and 12 or less, and even more preferably 5.0 or more and 10 or less.
<30> The aqueous composition for body according to any one of <1> to <29>, wherein a ratio of the number of moles of cationic charges of the component (A) to the number of moles of anionic charges of the component (B) in the aqueous composition for body is preferably 10/90 to 95/5, more preferably 20/80 to 95/5, and even more preferably 25/75 to 90/10.
<31> The aqueous composition for body according to any one of <1> to <30>, wherein a content of the component (C) in the aqueous composition for body is preferably 0.005 mass% or more and 5.0 mass% or less, more preferably 0.01 mass% or more and 4.0 mass% or less, even more preferably 0.02 mass% or more and 3.0 mass% or less, and even more preferably 0.02 mass% or more and 2.0 mass% or less.
<32> The aqueous composition for body according to any one of <1> to <31>, wherein a content of the component (C1) in the aqueous composition for body is preferably 0.02 mass% or more and 5.0 mass% or less, more preferably 0.04 mass% or more and 4.0 mass% or less, even more preferably 0.05 mass% or more and 3.0 mass% or less, even more preferably 0.1 mass% or more and 2.0 mass% or less, even more preferably 0.2 mass% or more and 2.0 mass% or less, and even more preferably 0.3 mass% or more and 2.0 mass% or less.
<33> The aqueous composition for body according to any one of <1> to <32>, wherein a content of the component (C2) in the aqueous composition for body is preferably 0.005 mass% or more and 1.0 mass% or less, more preferably 0.01 mass% or more and 0.5 mass% or less, even more preferably 0.01 mass% or more and 0.2 mass% or less, and even more preferably 0.02 mass% or more and 0.1 mass% or less.
<34> The aqueous composition for body according to any one of <1> to <33>, wherein a mass ratio [(C)/{(A) + (B)}] of the component (C) to a total amount of the component (A) and the component (B) in the aqueous composition for body is preferably 0.01 or more and 20 or less, more preferably 0.02 or more and 15 or less, even more preferably 0.02 or more and 10 or less, even more preferably 0.02 or more and 8.0 or less, even more preferably 0.02 or more and 5.0 or less, even more preferably 0.02 or more and 2.0 or less, and even more preferably 0.03 or more and 1.5 or less.
<35> The aqueous composition for body according to any one of <1> to <34>, wherein a mass ratio [(C1)/{(A) + (B)}] of the component (C1) to a total amount of the component (A) and the component (B) in the aqueous composition for body is preferably 0.2 or more and 20 or less, more preferably 0.3 or more and 15 or less, even more preferably 0.4 or more and 10 or less, even more preferably 0.5 or more and 8.0 or less, even more preferably 0.6 or more and 5.0 or less, even more preferably 0.8 or more and 2.0 or less, and even more preferably 0.8 or more and 1.5 or less.
<36> The aqueous composition for body according to any one of <1> to <35>, wherein a mass ratio [(C2)/{(A) + (B)}] of the component (C2) to a total amount of the component (A) and the component (B) in the aqueous composition for body is preferably 0.01 or more and 1.0 or less, more preferably 0.02 or more and 0.5 or less, even more preferably 0.02 or more and 0.3 or less, and even more preferably 0.03 or more and 0.2 or less.
<37> The aqueous composition for body according to any one of <1> to <36>, wherein a content of water in the aqueous composition for body is preferably 50 mass% or more, more preferably 70 mass% or more, even more preferably 80 mass% or more, even more preferably 90 mass% or more.
<38> The aqueous composition for body according to any one of <1> to <37>, wherein an aqueous solution prepared by 20-fold dilution of the aqueous composition for body with water has a pH at 25°C of preferably 3.0 or more, more preferably 3.2 or more, and preferably 7.5 or less, more preferably 7.0 or less, even more preferably 6.0 or less, and even more preferably 5.0 or less.
<39> A method for producing the aqueous composition for body described in any one of <1> to <38>, the method including dissolving or dispersing the component (B) and the component (C) in water to prepare a liquid mixture containing the component (B) and the component (C), and then mixing the liquid mixture with the component (A) or an aqueous solution thereof.
<40> A cosmetic composition containing the aqueous composition for body described in any one of <1> to <39>.
<41> The cosmetic composition according to <40>, wherein a content of the aqueous composition for body in the cosmetic composition is 1 mass% or more and 80 mass% or less, preferably 10 mass% or more and 60 mass% or less.
<42> The cosmetic composition according to <40> or <41>, wherein a content of the component (A) in the cosmetic composition is preferably 0.001 mass% or more and 3.0 mass% or less, more preferably 0.005 mass% or more and 2.0 mass% or less, even more preferably 0.01 mass% or more and 1.5 mass% or less, even more preferably 0.05 mass% or more and 1.5 mass% or less, and even more preferably 0.05 mass% or more and 1.0 mass% or less.
<43> The cosmetic composition according to any one of <40> to <42>, wherein a content of the component (B) in the cosmetic composition is preferably 0.0005 mass% or more and 3.0 mass% or less, more preferably 0.001 mass% or more and 2.0 mass% or less, even more preferably 0.01 mass% or more and 1.5 mass% or less, even more preferably 0.02 mass% or more and 1.0 mass% or less, even more preferably 0.02 mass% or more and 0.5 mass% or less, even more preferably 0.02 mass% or more and 0.3 mass% or less, even more preferably 0.02 mass% or more and 0.2 mass% or less, and even more preferably 0.02 mass% or more and 0.1 mass% or less.
<44> The cosmetic composition according to any one of <40> to <43>, wherein a total content of the component (A) and the component (B) in the cosmetic composition is preferably 0.0015 mass% or more and 5.0 mass% or less, more preferably 0.01 mass% or more and 4.0 mass% or less, even more preferably 0.02 mass% or more and 4.0 mass% or less, even more preferably 0.05 mass% or more and 2.0 mass% or less, and even more preferably 0.05 mass% or more and 1.5 mass% or less.
<45> The cosmetic composition according to any one of <40> to <44>, wherein a content of the component (C) in the cosmetic composition is preferably 0.001 mass% or more and 5.0 mass% or less, more preferably 0.002 mass% or more and 4.0 mass% or less, even more preferably 0.005 mass% or more and 4.0 mass% or less, even more preferably 0.005 mass% or more and 2.0 mass% or less, and even more preferably 0.01 mass% or more and 1.5 mass% or less.
<46> A hair treatment method including applying the cosmetic composition described in any one of <40> to <45> to hair, and then heating the hair to a temperature of 80°C or more.
<47> The hair treatment method according to <46>, wherein the amount of the cosmetic composition applied to the hair is preferably 0.005 or more, more preferably 0.01 or more, even more preferably 0.05 or more, and preferably 20 or less, more preferably 15 or less, even more preferably 12 or less in terms of a bath ratio (mass of the cosmetic composition/dry mass of the hair to which the cosmetic composition is applied).
<48> The hair treatment method according to <46> or <47>, wherein the cosmetic composition is washed off with water after application of the cosmetic composition to hair and before heating of the hair.
<49> The hair treatment method according to any one of <46> to <48>, wherein a heating temperature of the hair is preferably 100°C or more, more preferably 120°C or more, and even more preferably 140°C or more, and preferably 220°C or lower, and more preferably 200°C or lower.

### Examples

The present invention will be described below through examples, but the present invention is not limited to the scope of the examples. Each measurement and evaluation in Examples were performed by the following methods.

### (Measurement of Viscosity of 1 mass% Aqueous Solution of Cationic Polymer)

A 1 mass% aqueous solution of the active ingredient of the cationic polymer (component (A) or component (A')) used in each example was prepared, and the aqueous solution viscosity at 30°C was measured using a B-type viscometer (TVB-10 manufactured by Toki Sangyo Co., Ltd.). In the present example, the cationic polymer other than the component (A) was denoted as "component (A')".

### (pH Measurement)

The aqueous composition of each example was diluted by 20 times with water, and the pH at 25°C was measured using a pH meter (F-51, manufactured by Horiba, Ltd.).

### (Preparation of Damaged Tress)

"Kao Foam Hair Bleach L" (first agent) 7 mL and "Kao Foam Hair Color d" (second agent) 15 mL manufactured by Kao Corporation were mixed, and the mixture was applied to a tress of Japanese hair having a length of 20 cm and a mass of 20 g. After being left to stand for 20 minutes, it was sufficiently rinsed with warm water at 35 to 40°C (bleaching treatment). Thereafter, the tress was washed twice with a plain shampoo having the following composition. The bleaching treatment was repeated four times to prepare a damaged tress for evaluation.

### (Measurement of Combing Force Immediately After Treatment with Aqueous Composition)

(1) The damaged tress in an amount of 20 g was impregnated with 10 g ± 0.5 g of water, the aqueous composition of each example was applied in an amount of 4 mL, spread by hand, left for 30 seconds, and then rinsed with warm water at 35 to 40°C for 15 seconds.
(2) The water content of the tress after rinsing was adjusted to 10 g, the tress was combed with a brush to completely remove the entanglement, and then the tress was set in a combing force measuring device ("KOT-0303" manufactured by UTSUNOMIYA SEIKI Co., Ltd.). A commercial hairbrush SL-801 (manufactured by SANBI KOGYO K.K.) was passed through the top of the tress to manually comb the tress at a constant speed (60 rpm), and the load (g) applied when the tress was passed to the hair tip was measured. Combing was performed 50 times, and the average value of the combing loads at 50 points in total was taken as the value of "Combing force immediately after treatment". A smaller value of the combing force indicates that hair is less likely to get entangled and a better result is obtained. The combing load of the damage trace (containing 10 g ± 0.5 g of water) which was not treated with the aqueous composition was 1568 g.

### [Plain Shampoo Composition]

| | (mass%) |
|---|---|
| Sodium polyoxyethylene (2.0) lauryl ether sulfate (*1) | : 15.5 |
| Diethanolamide laurate (*2) | : 2.2 |
| Disodium edetate | : 0.15 |
| Sodium benzoate | : 0.18 |
| Oxybenzone | : 0.03 |
| Phosphoric acid | : 0.07 |
| Sodium chloride | : 0.8 |
| Fragrance | : 0.4 |
| Purified water | : Balance |
| Total | : 100.00 |

| | |
|---|---|
| (*1) 42.0 mass% as "EMAL E-27C" (manufactured by Kao Corporation, active ingredient amount 27 mass%) (*2) AMINON C-11S (manufactured by Kao Corporation) | |

### (Measurement of Combing Force After Treatment with Aqueous Composition + Two Times of Hair Washing)

After performing (1) to (2) in the "Measurement of Combing Force Immediately After Treatment", the following (3) was performed twice.

(3) After towel drying and dryer drying, the tress was wetted again with water, and the front and back of the tress was scrubbed 20 times each for 30 seconds with 1 mL of the plain shampoo and rinsed for 15 seconds.

Thereafter, the operation of (2) was performed, and the combing force was measured. When the combing force was equal to or less than the 850 g, the durability was determined to be good.

### (Measurement of Combing Force After Treatment with Aqueous Composition + Four Times of Hair Washing)

In the above "Measurement of Combing Force Immediately After Treatment ", (1) to (2) were performed, thereafter (3) was performed four times, and then the operation of (2) was performed again to measure the combing force. When the combing force value was smaller, the durability was better, and when the combing force was equal to or less than the 950 g, the durability was determined to be better.

### (Measurement of Combing Force After Treatment with Aqueous Composition + Heating Treatment + Four Times of Hair Washing)

After performing (1) and (2) in the "Measurement of Combing Force Immediately After Treatment", towel drying and dryer drying of the tress were performed. Next, straight setting was performed 10 times with a flat iron ("VOSS GF (L41)" manufactured by GOLDWELL) set at 180°C while applying tension with a comb (heating treatment). After the heating treatment, (3) was performed four times and then the operation of (2) was performed to measure the combing force.

### Examples 1 to 21 and Comparative Examples 1 to 14 (Preparation and Evaluation of Aqueous Composition)

Aqueous compositions were prepared by the following procedure using the components listed in the table.

A 2 mass% aqueous solution of a cationic polymer (component (A) or component (A')), a 2 mass% aqueous solution of an anionic polymer (B1), a 1 mass% aqueous solution of an anionic polymer (B2), and a 5 mass% aqueous solution of a nonionic polymer (C1) were prepared. These aqueous solutions, the other components shown in the table, and the remaining amount of water were mixed so that each component became the blending amount shown in the table. Specifically, water, the aqueous solution of (B1) an anionic polymer, the aqueous solution of (C1) a nonionic polymer, the aqueous solution of (B2) an anionic polymer, and an acid (salt in Comparative Examples 12 to 14) were added in this order, and stirred with a pencil mixer. While stirring was continued, the aqueous solution of the cationic polymer (component (A) or component (A')) was gradually added to obtain an aqueous composition.

The obtained aqueous composition was evaluated by the above-described method. The results are shown in Tables 1 to 6.

### Table 1

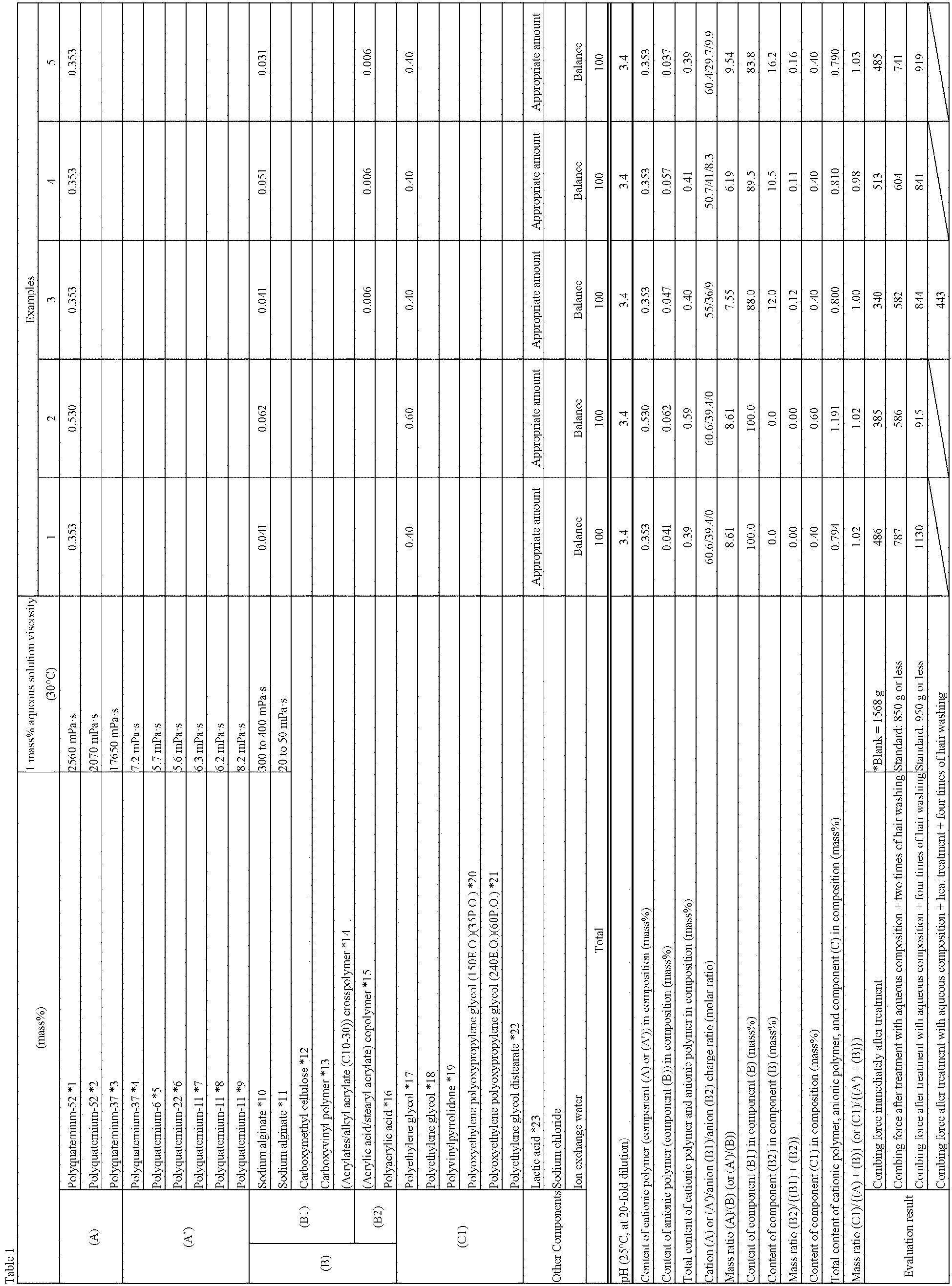

### Table 2

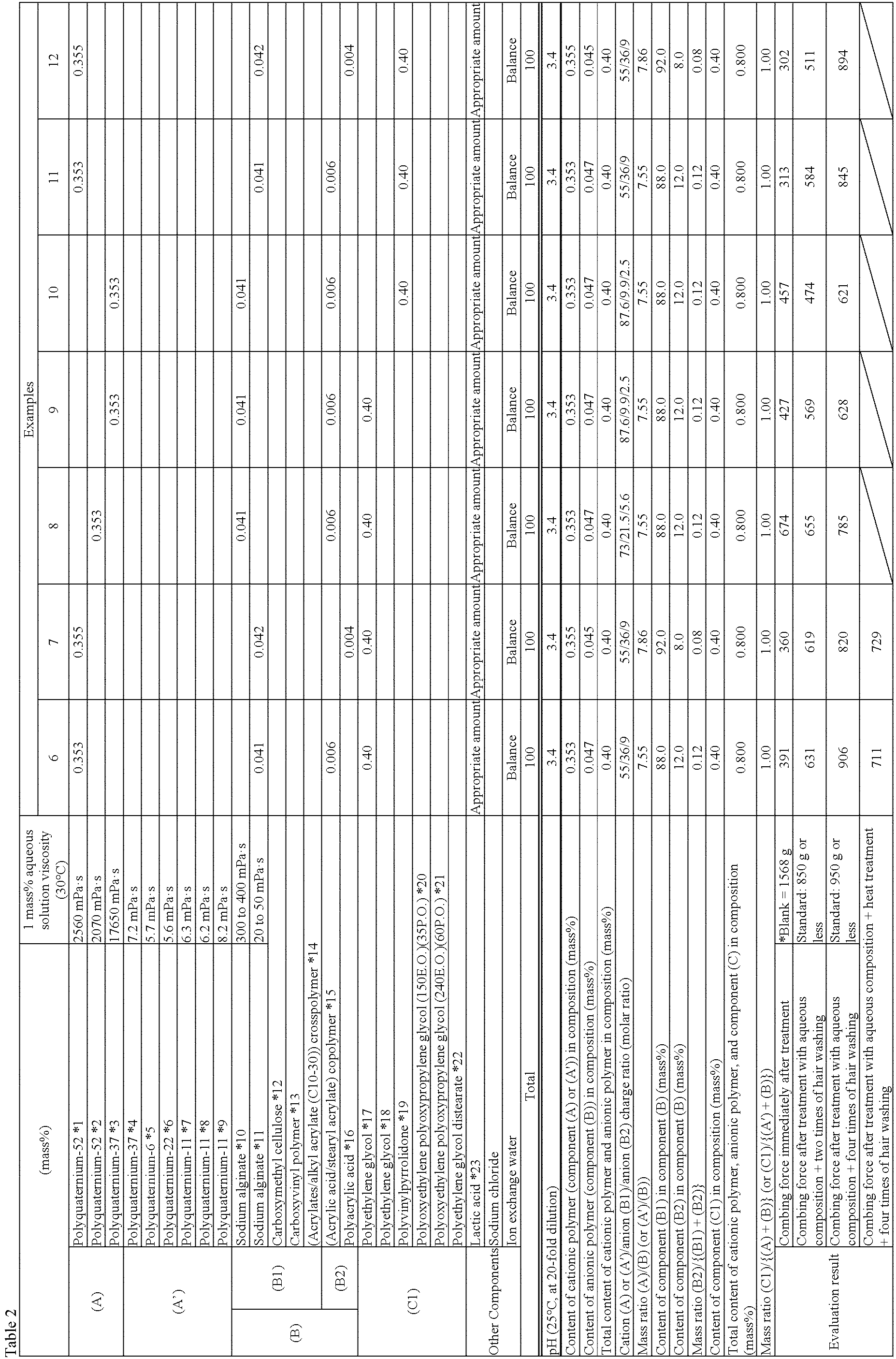

### Table 3

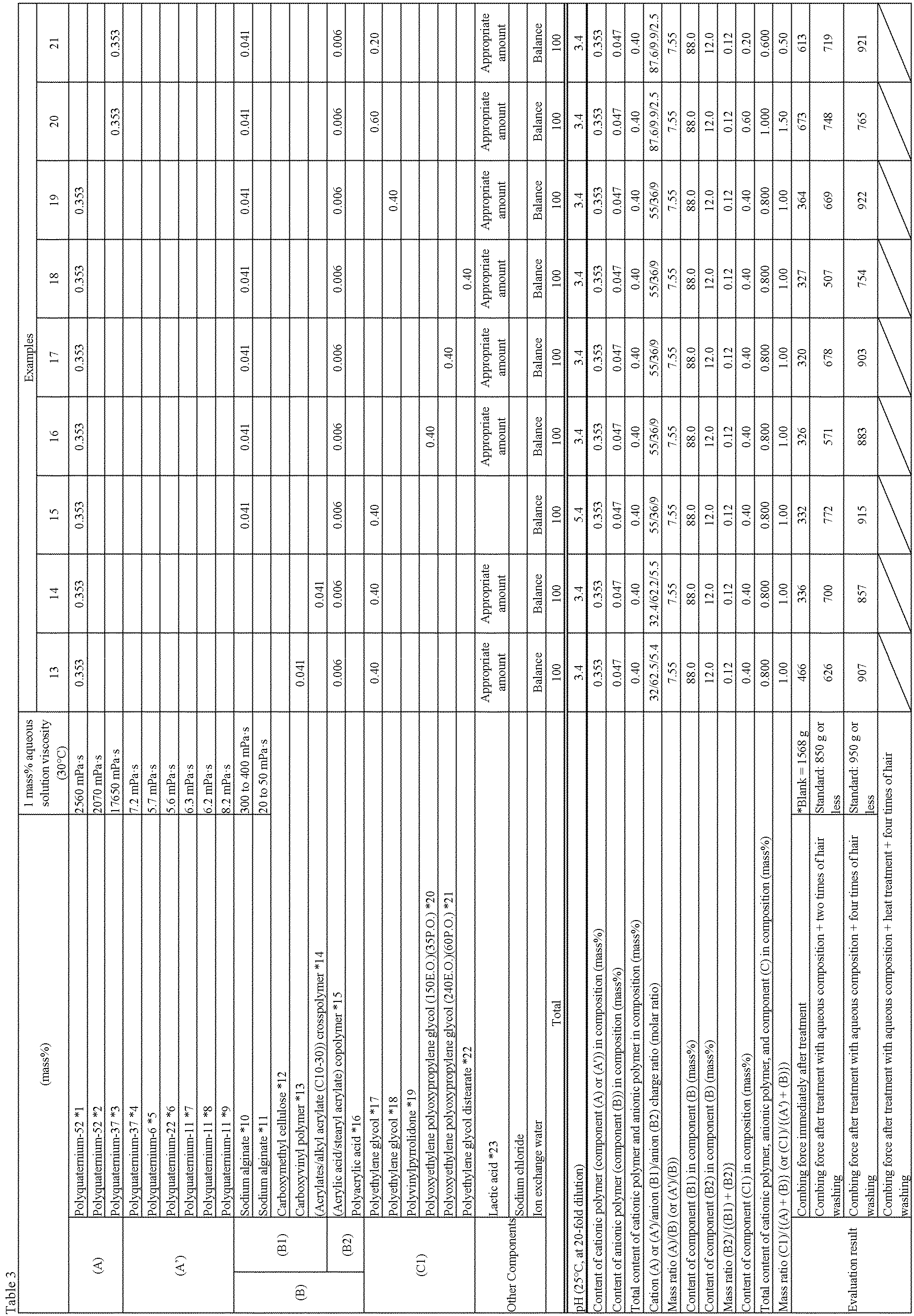

### [Table 4]

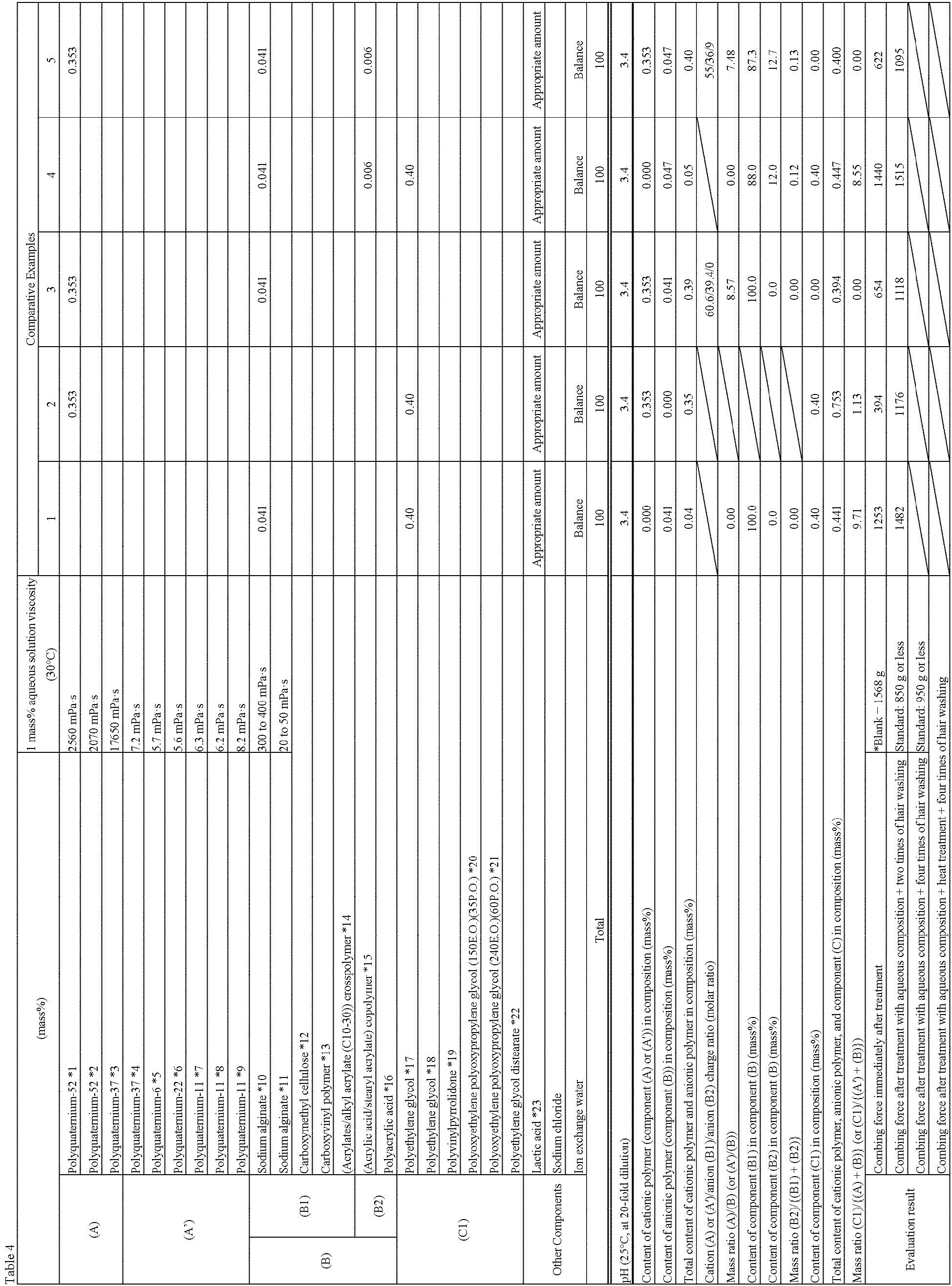

### [Table 5]

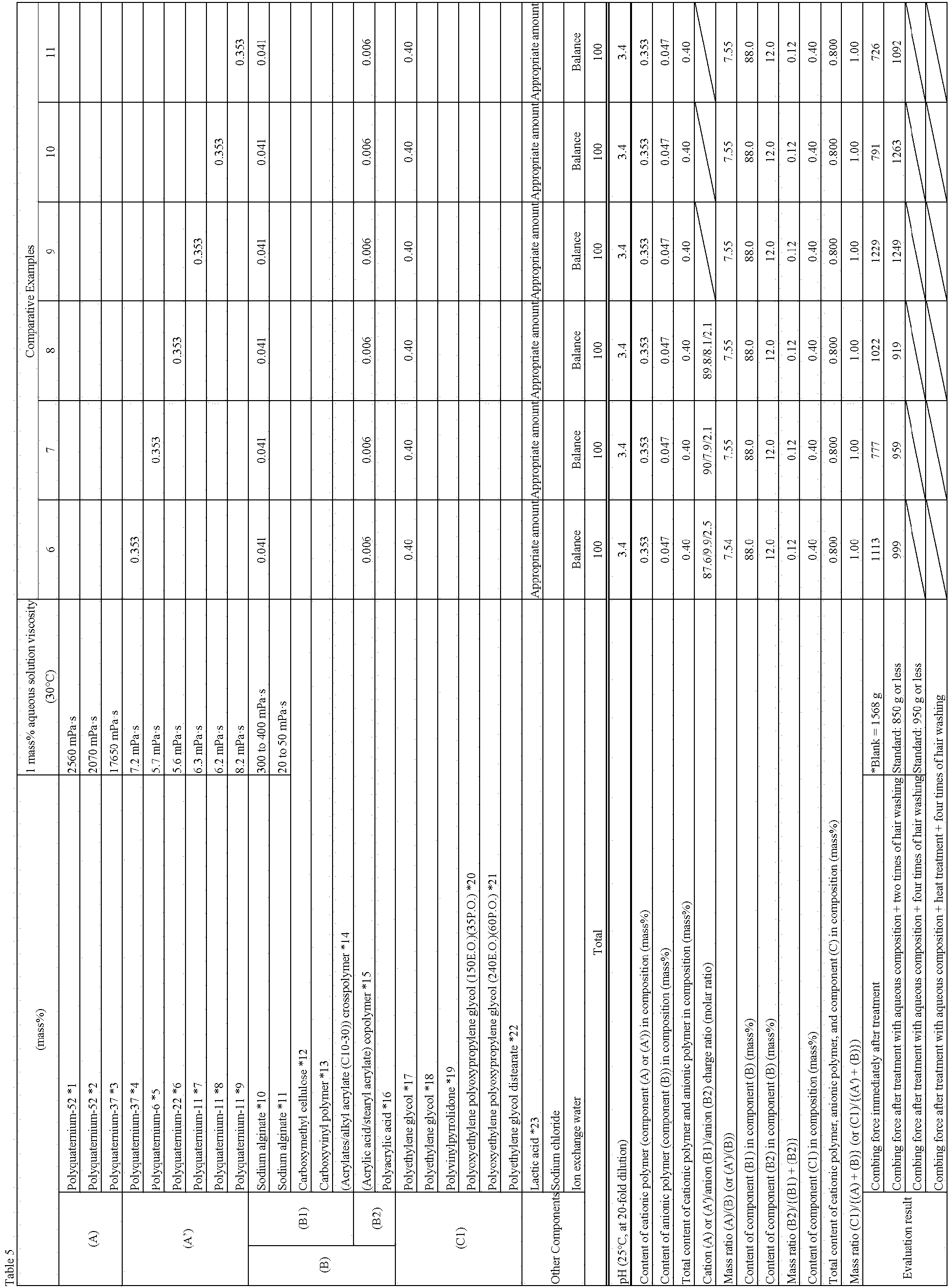

### [Table 6]

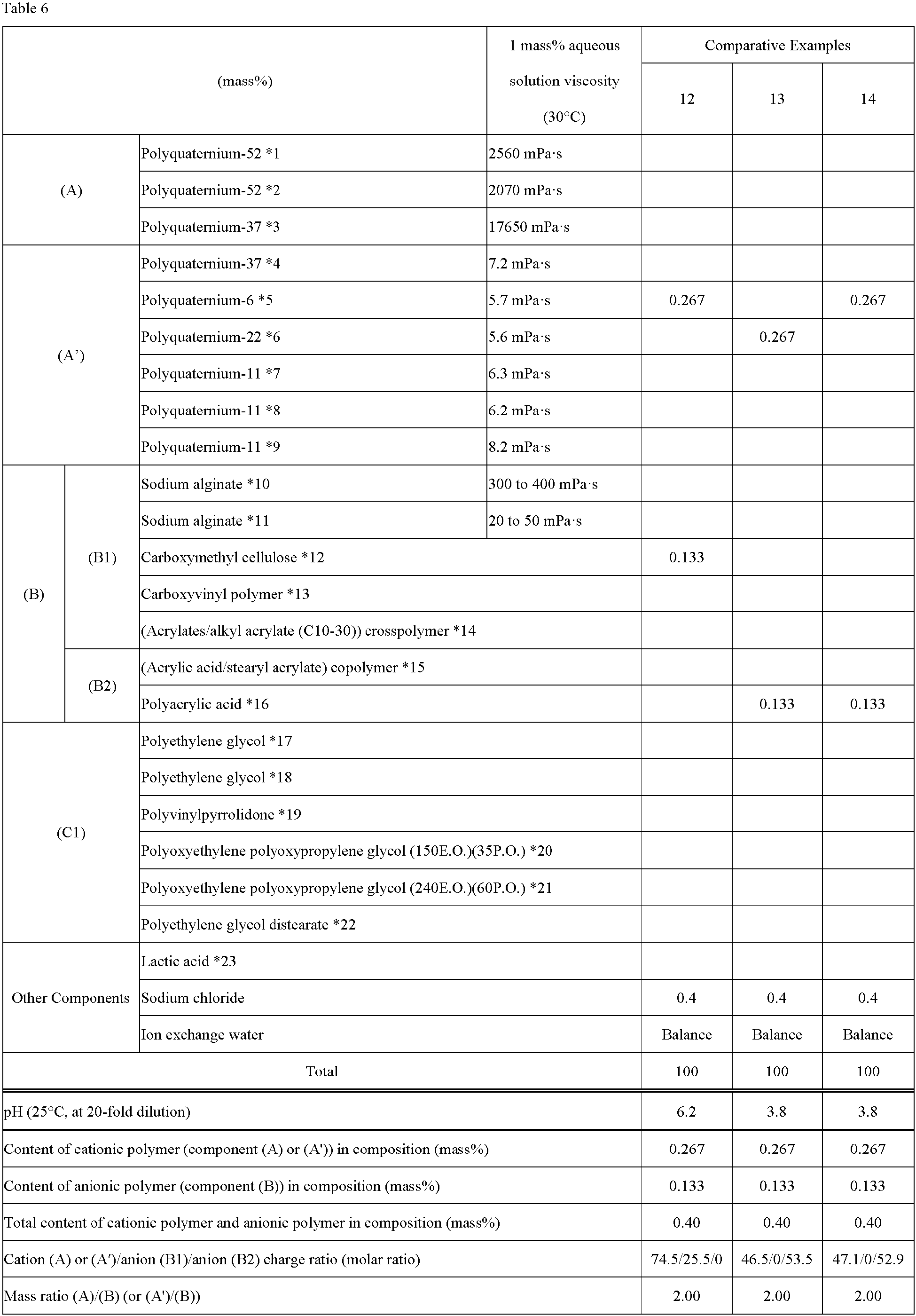

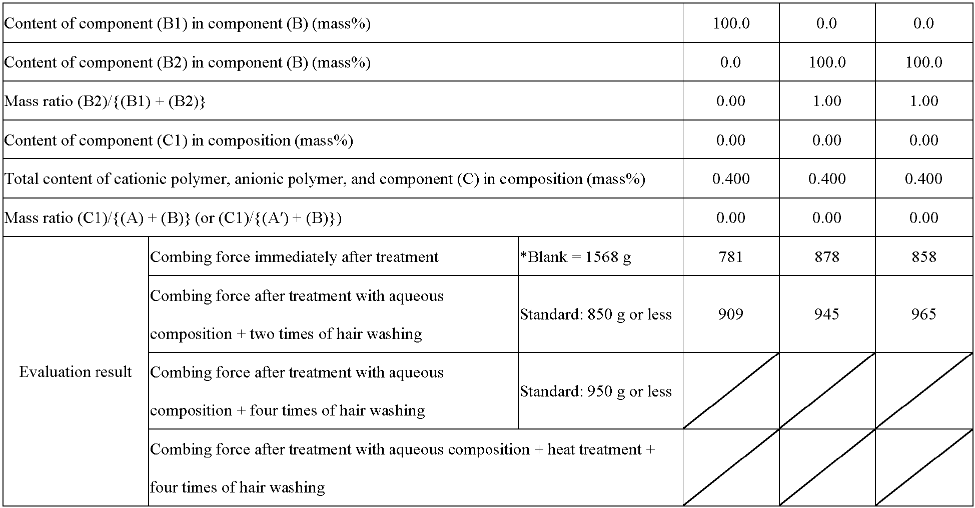

### Examples 22 to 26 and Comparative Examples 15 to 18 (Preparation and Evaluation of Aqueous Composition)

Aqueous compositions were prepared by the following procedure using the components listed in the table.

A 2 mass% aqueous solution of a cationic polymer (component (A) or component (A')), a 2 mass% aqueous solution of an anionic polymer (B1), and a 1 mass% aqueous solution of agar (gel) (C2) were prepared. These aqueous solutions, the other components shown in the table, and the remaining amount of water were mixed so that each component became the blending amount shown in the table. Specifically, water, the aqueous solution of a cationic polymer (component (A) or component (A')), the aqueous solution of agar (C2), and an acid were added in this order, and the mixture was stirred with a stirring blade while being heated in a water bath at 90°C until the agar was completely dissolved. Once the agar had dissolved, the aqueous solution of (B1) an anionic polymer was added slowly while continuing heating at 90°C. After completion of the addition, heating was stopped, and the mixture was allowed to cool to normal temperature while being stirred to obtain an aqueous composition.

The obtained aqueous composition was evaluated by the above-described method. The results are shown in Tables 7 and 8.

### Examples 27 to 30

Aqueous compositions were prepared by the following procedure using the components listed in the table.

A 2 mass% aqueous solution of a cationic polymer (component (A) or component (A')), a 2 mass% aqueous solution of an anionic polymer (B1), a 1 mass% aqueous solution of an anionic polymer (B2), a 1 mass% aqueous solution of agar (C2) (gel), and a 5 mass% aqueous solution of a nonionic polymer (C1) were prepared. These aqueous solutions, the other components, and the remaining amount of water were mixed so that each component became the blending amount shown in the table. Specifically, water, the aqueous solution of (B1) an anionic polymer, the aqueous solution of (C1) a nonionic polymer, the aqueous solution of (B2) an anionic polymer, an acid, and the aqueous solution of (C2) agar were added in this order, and the mixture was stirred with a stirring blade while being heated in a water bath at 90°C until the (C2) agar was completely dissolved. Once the agar (C2) had dissolved, the aqueous solution of cationic polymer (component (A) or component (A')) was added slowly while continuing heating at 90°C. After completion of the addition, heating was stopped, and the mixture was allowed to cool to normal temperature while being stirred to obtain an aqueous composition.

The obtained aqueous composition was evaluated by the above-described method. The results are shown in Table 7.

### Comparative Examples 19 to 21

Aqueous compositions were prepared by the following procedure using the components listed in the table.

A 2 mass% aqueous solution of a cationic polymer (component (A')), a 2 mass% aqueous solution of an anionic polymer (B1), and a 1 mass% aqueous solution of an anionic polymer (B2) were prepared. These aqueous solutions, the other components shown in the table, and the remaining amount of water were mixed so that each component became the blending amount shown in the table. Specifically, water, the aqueous solution of (B1) an anionic polymer, the aqueous solution of (B2) an anionic polymer, and a salt were added in this order, and these were stirred with a pencil mixer. While stirring was continued, the aqueous solution of the cationic polymer (component (A')) was gradually added to obtain an aqueous composition.

The obtained aqueous composition was evaluated by the above-described method. The results are shown in Table 8.

### [Table 7]

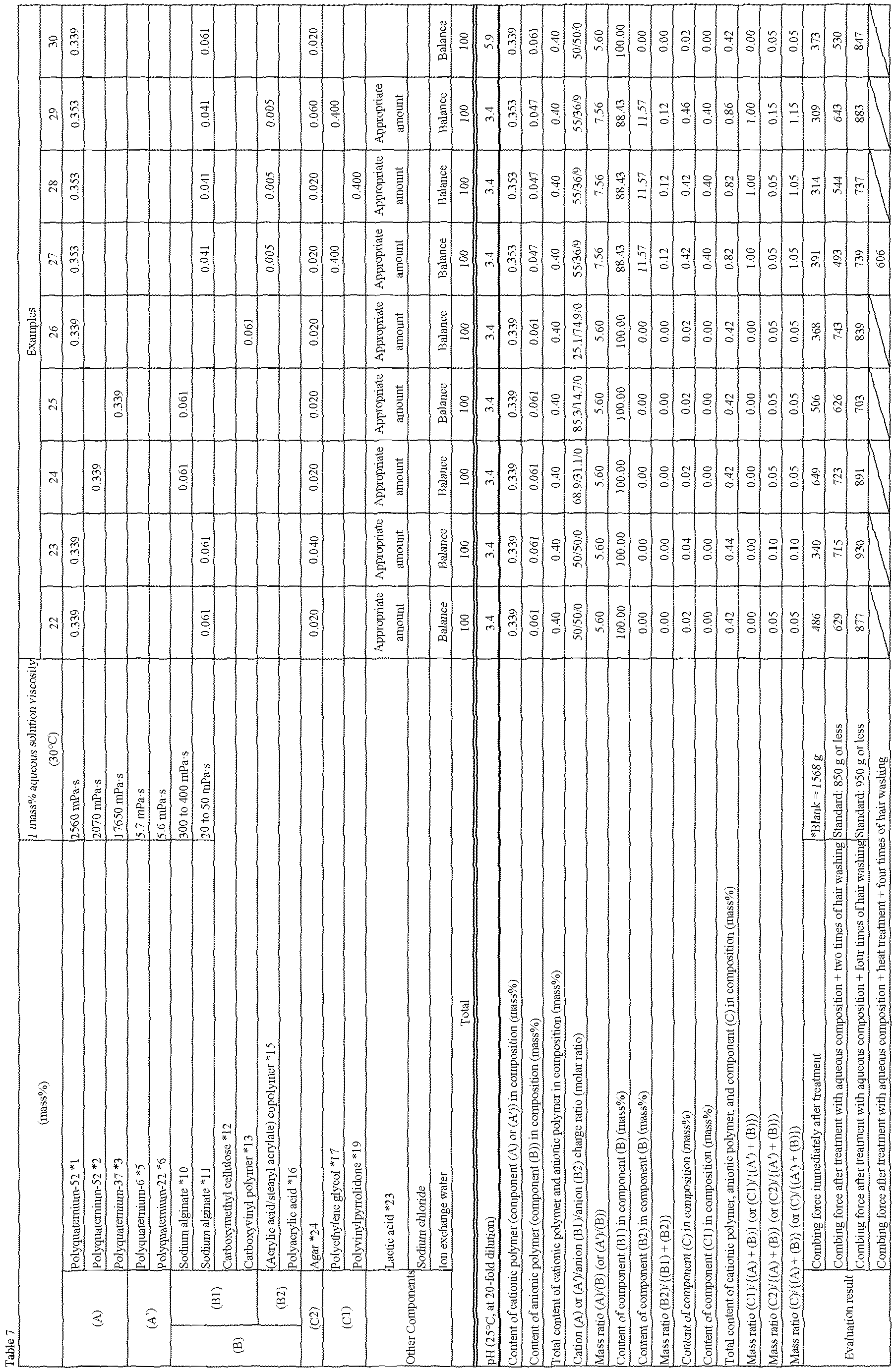

### [Table 8]

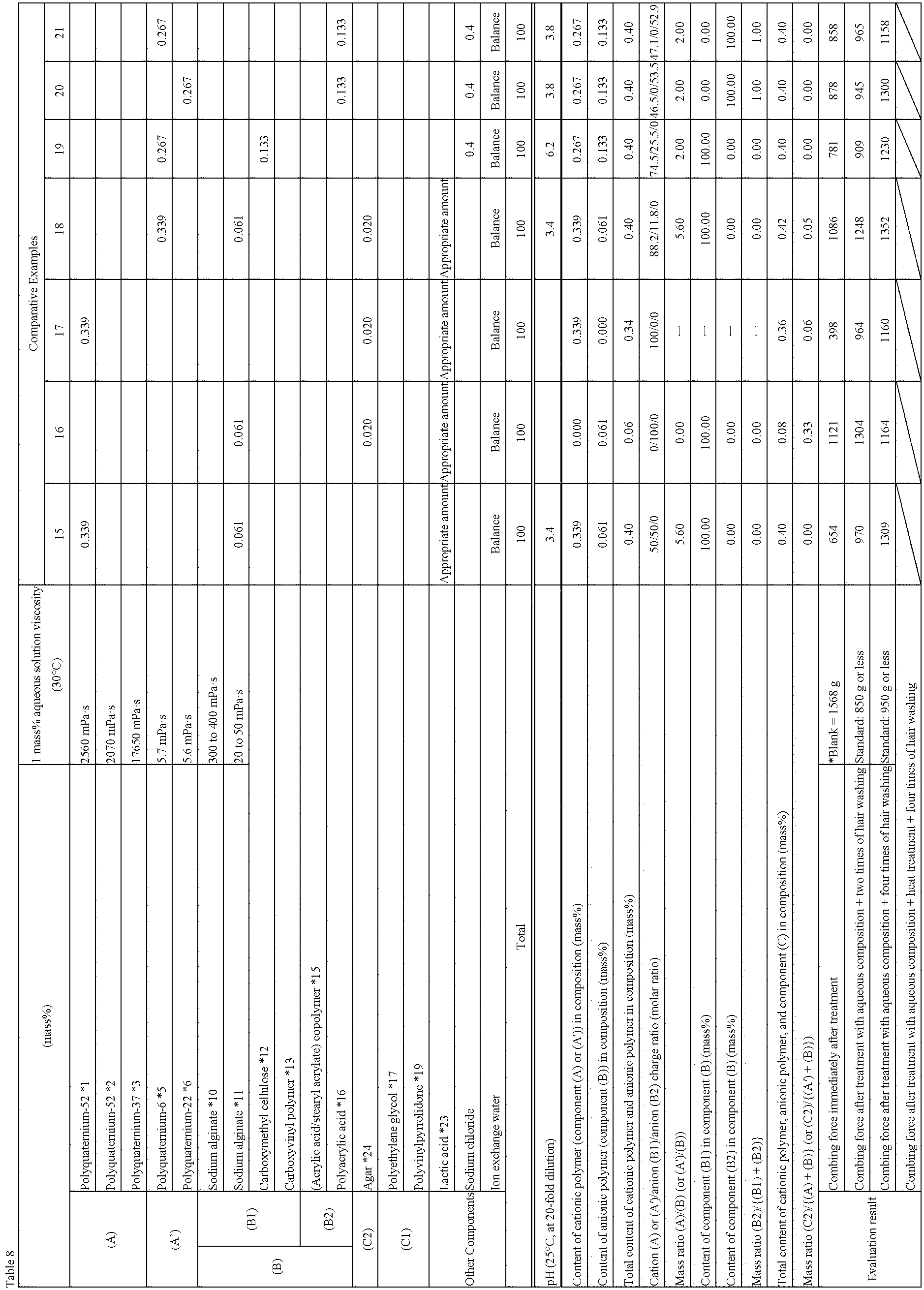

The components shown in Tables 1 to 8 are as follows. The blending amount (mass%) described in the table is an effective amount of each component.

### <Component (A): Cationic Polymer>

*1 Polyquaternium-52 (N,N-dimethylaminoethyl methacrylate diethyl sulfate-N,N-dimethylacrylamide-polyethylene glycol dimethacrylate copolymer), "SOFCARE KG-101W-E" manufactured by Kao Corporation, charge density 0.83 mmol/g (active ingredient amount 2.4 mass%)
*2 Polyquaternium-52 (N,N-dimethylaminoethyl methacrylate diethyl sulfate-N,N-dimethylacrylamide-polyethylene glycol dimethacrylate copolymer), "SOFCARE KG-301W" manufactured by Kao Corporation, charge density 1.84 mmol/g (active ingredient amount 4.02 mass%)
*3 Polyquaternium-37 (methacryloylethyltrimethylammonium chloride polymer), "Cosmedia Ultragel 300" manufactured by BASF Japan Ltd., content of constituent unit represented by General Formula (1): 100 mol%, charge density: 4.81 mmol/g (active ingredient amount 96.9 mass%)

### <Component (A'): Cationic Polymer Other Than Component (A)>

*4 Polyquaternium-37 (methacryloylethyltrimethylammonium chloride polymer), "KP polymer E" manufactured by Kao Corporation, charge density 4.81 mmol/g (active ingredient amount 35 mass%)
* 5 Polyquaternium-6 (dimethyldiallylammonium chloride polymer), "Merquat 100" manufactured by Lubrizol Advanced Materials, Inc., charge density 6.18 mmol/g (active ingredient amount 41.6 mass%)
*6 Polyquaternium-22 (dimethyldiallylammonium chloride-acrylic acid copolymer liquid), "Merquat 295" manufactured by Lubrizol Advanced Materials, Inc., charge density 6.04 mmol/g

### (active ingredient amount 37 mass%)

*7 Polyquaternium-11 (vinylpyrrolidone-N,N-dimethylaminoethyl methacrylate copolymer diethyl sulfate), "GAFQUAT 440 " manufactured by Ashland Japan K.K. (active ingredient amount 30 mass%)
*8 Polyquaternium-11 (vinylpyrrolidone-N,N-dimethylaminoethyl methacrylate copolymer diethyl sulfate), "GAFQUAT 734" manufactured by Ashland Japan K.K. (active ingredient amount 50 mass%)
*9 Polyquaternium-11 (vinylpyrrolidone-N,N-dimethylaminoethyl methacrylate copolymer diethyl sulfate), "GAFQUAT 755N" manufactured by Ashland Japan K.K. (active ingredient amount 20 mass%)

### <Component (B1): Anionic Polymer>

*10 Sodium alginate, "KIMICA ALGIN I-3-150 " manufactured by KIMICA Corporation, Mw: 1900000 to 2300000, charge density 4.65 mmol/g
*11 Sodium alginate, "KIMICA ALGIN IL-2" manufactured by KIMICA Corporation, Mw: 500000 to 900000, charge density 4.65 mmol/g
*12 Carboxymethyl cellulose, "CELLOGEN F-5" manufactured by Dai-ichi Kogyo Seiyaku Co., Ltd., charge density 4.23 mmol/g
*13 Carboxyvinyl polymer, "Carbopol 980" manufactured by Lubrizol Advanced Materials, Inc., charge density 13.88 mmol/g
*14 (Acrylates/alkyl acrylate (C10-30)) crosspolymer, "Pemulen TR-1" manufactured by Lubrizol Advanced Materials, Inc., charge density 13.67 mmol/g

### <Component (B2): Anionic Polymer>

*15 (Acrylate/stearyl acrylate) copolymer, "SOFCARE SA-37W" manufactured by Kao Corporation, Mw 20000, charge density 8.88 mmol/g (active ingredient amount 10 mass%)
*16 Polyacrylic acid "JURYMER AC-10L" manufactured by Toagosei Co., Ltd., Mw 20000 to 30000, charge density 13.88 mmol/g (active ingredient amount 40 mass%)

### <Component (C1): Nonionic polymer>

*17 Polyethylene glycol, "BLAUNON PEG-20000S" manufactured by AOKI OIL INDUSTRIAL Co., Ltd., Mw: 20000
*18 Polyethylene glycol, "Wako polyethylene glycol 6000" manufactured by FUJIFILM Wako Pure Chemical Industries, Ltd., Mw: 6000
*19 Polyvinylpyrrolidone, "Luviskol K90" manufactured by BASF Japan Ltd., Mw: 1200000
*20 Polyoxyethylene polyoxypropylene glycol (150E.O.)(35P.O.), "PRONON #208" manufactured by NOF Corporation
*21 Polyoxyethylene polyoxypropylene glycol (240E.O.)(60P.O.), "UNILUBE 75DE-2620R" manufactured by NOF Corporation
*22 Polyethylene glycol distearate "EMULMIN 862" manufactured by Sanyo Chemical Industries, Ltd.

### <Acid>

*23 90% Lactic acid, "PURAC ULTRAPURE90" manufactured by PURAC Thailand Ltd. (active ingredient amount 90 mass%)

### <Component (C2): Agar>

*24 Reagent WAKO agar, manufactured by FUJIFILM Wako Pure Chemical Industries, Ltd., jelly strength: 400 to 600 g/cm²

As shown in Tables 1 to 8, it can be seen that the hair treated with the aqueous composition of the present Example is hardly entangled, and the treatment effect is maintained even after hair washing. In addition, from the results of Examples 3, 6, and 7, it can be seen that when the step of heating the hair treated with the aqueous composition of the present Example to a temperature of 80°C or more is performed, the combing force after four times of hair washing is lower than that in the case where the heat treatment is not performed, and the effect of suppressing entanglement of hair is improved.

### Industrial Applicability

According to the present invention, it is possible to provide an aqueous composition for body that can improve the effect of improving the texture of an object to be treated and the durability of the effect, and in particular, can improve the effect of suppressing entanglement of hair and the durability thereof when used for hair treatment.

The aqueous composition can be used as various cosmetic compositions such as a skin cosmetic composition and a hair cosmetic composition. The aqueous composition can also be used as a premix for various cosmetic compositions. For example, additional components may be added to the aqueous composition to prepare a skin cosmetic composition or a hair cosmetic composition.

## Claims

1. An aqueous composition for body, the aqueous composition comprising (A) a cationic polymer having a viscosity when in a 1 mass% aqueous solution form at 30°C of 1000 mPa·s or more, (B) an anionic polymer, (C) one or more selected from the group consisting of (C1) a nonionic polymer and (C2) agar, and water.

2. The aqueous composition for body according to claim 1, wherein the component (A) is a polymer comprising a constituent unit represented by the following General Formula (1): 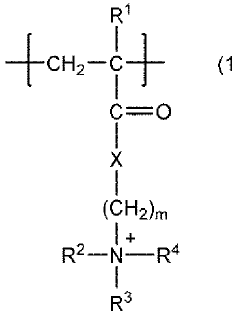 wherein R¹ is a hydrogen atom or a methyl group, R² to R⁴ are each independently an alkyl group having 1 or more and 4 or less carbons, X is -O- or -NH-, and m is the number of 1 or more and 4 or less.

3. The aqueous composition for body according to claim 1 or 2, wherein a mass ratio [(A)/(B)] of the component (A) to the component (B) contained in the aqueous composition for body is 1.0 or more and 30 or less.

4. The aqueous composition for body according to any one of claims 1 to 3, wherein a mass ratio [(C1)/{(A) + (B)}] of the component (C1) to a total amount of the component (A) and the component (B) contained in the aqueous composition for body is 0.2 or more and 20 or less.

5. The aqueous composition for body according to any one of claims 1 to 3, wherein a mass ratio [(C2)/{(A) + (B)}] of the component (C2) to a total amount of the component (A) and the component (B) contained in the aqueous composition for body is 0.01 or more and 1.0 or less.

6. The aqueous composition for body according to any one of claims 1 to 5, wherein the component (C1) is one or more selected from the group consisting of a polyalkylene glycol or a derivative thereof, and a polymer containing a constituent unit derived from vinylpyrrolidone.

7. The aqueous composition for body according to any one of claims 1 to 6, wherein the component (B) comprises (B1) one or more selected from the group consisting of a crosslinked polymer containing a constituent unit derived from (meth)acrylic acid and an anionic polysaccharide.

8. The aqueous composition for body according to claim 7, wherein the component (B1) is alginic acid or a salt thereof.

9. The aqueous composition for body according to claim 7 or 8, wherein the component (B) further comprises (B2) an anionic polymer other than the component (B1).

10. The aqueous composition for body according to claim 9, wherein the component (B2) is a non-crosslinked polymer having a molecular weight of 1000 or more and 30000 or less and containing a constituent unit derived from (meth)acrylic acid.

11. The aqueous composition for body according to any one of claims 1 to 10, wherein an aqueous solution prepared by 20-fold dilution of the aqueous composition for body with water has a pH of 5.0 or less at 25°C.

12. The aqueous composition for body according to any one of claims 1 to 11, wherein the component (A) is one or more selected from the group consisting of a methacryloylethyltrimethylammonium chloride polymer and an N,N-dimethylaminoethylmethacrylic acid diethyl sulfate-N,N-dimethylacrylamide-polyethylene glycol dimethacrylate copolymer.

13. A cosmetic composition comprising the aqueous composition for body described in any one of claims 1 to 12.

14. A hair treatment method comprising applying the cosmetic composition described in claim 13 to hair, and then heating the hair to a temperature of 80°C or more.
